# EUROPEAN PATENT APPLICATION

(11) **EP 4 332 120 A1**
(43) Date of publication of application: **06.03.2024**
(21) Application number: 22794892.4
(22) Date of filing: 26.04.2022
(51) Int. Cl.: C07K 16/46, A61P 35/00, C07K 16/28, C07K 5/027, A61K 47/68

(54) **BISPECIFIC ANTIBODY-DRUG CONJUGATE**

(30) Priority: 26.04.2021 CN 202110452948
(71) Applicant: Xuanzhu Biopharmaceutical Co., Ltd., Shijiazhuang, Hebei 050035 (CN); Beijing Xuanzhu Combio Co., Ltd., Beijing 100176 (CN)
(72) Inventor: ZHU, Xiaodong, Beijing 100176 (CN); YE, Jintong, Beijing 100176 (CN); WANG, Jinfeng, Beijing 100176 (CN); HE, Wei, Beijing 100176 (CN)
(74) Representative: J A Kemp LLP
(86) International application number: PCT/CN2022/089229
(87) International publication number: WO 2022/228422

(57) **Abstract**

The present invention relates to a bispecific antibody-drug conjugate comprising a bispecific antibody, a toxin, and a linker, and to a pharmaceutical composition comprising the bispecific antibody-drug conjugate, and a use of the bispecific antibody-drug conjugate and the pharmaceutical composition thereof in the preparation of medicaments for preventing and/or treating cancer. The bispecific antibody comprises a first paratope capable of recognizing and/or binding to domain II of HER2, and a second paratope capable of recognizing and/or binding to domain IV of HER2; the toxin is selected from the group consisting of maytansines, hemiasterlins, amanitins, auristatins, calicheamicins and duocarmycins; the linker is a cleavable linker or a non-cleavable linker; alternatively, one or more hydrogen atoms in the toxin and/or linker are optionally deuterated.

## Description

### TECHNICAL FIELD

The present invention belongs to the technical field of antibody drugs and specifically relates to a bispecific antibody-drug conjugate, a pharmaceutical composition comprising the bispecific antibody-drug conjugate, and a use of the bispecific antibody-drug conjugate and the pharmaceutical composition thereof in the preparation of medicaments for preventing and/or treating cancer.

### BACKGROUND

Human epidermal growth factor receptor 2 (HER2, also known as ErbB2) is a member of the ErbB family of tyrosine protein kinase receptors. It is a type I membrane protein with a single-channel transmembrane domain, an extracellular domain and a cytoplasmic kinase domain. The HER2 gene is amplified (HER2⁺) in approximately 20% of breast cancer patients. Targeting HER2 with monoclonal antibodies has been shown to be very effective in treating HER2-amplified breast cancer patients.

Trastuzumab (mAb 4D5, Herceptin^{®}) is a humanized anti-HER2 monoclonal antibody, which binds to domain IV of HER2 ECD and was approved by the FDA in 1998 as a treatment for HER2⁺ breast cancer.

Pertuzumab (rhuMab 2C4, PERJETA^{®}) is another humanized anti-HER2 monoclonal antibody, which binds to domain II of HER2 ECD. Domain II is a separate epitope on HER2 from that for trastuzumab. As domain II of HER2 ECD is involved in dimerization, the binding of pertuzumab to HER2 prevents HER2 from dimerizing with another receptor (such as EGFR, HER3 or HER4).

The combination of pertuzumab and trastuzumab shows superior efficacy to trastuzumab or pertuzumab alone, and has been approved by the FDA as a treatment for HER2⁺ metastatic breast cancer (2012) and one year later as a neoadjuvant treatment for HER2⁺ breast cancer (2013).

The international patent publication WO2018191188A1 discloses a bispecific antibody with a common light chain that targets both domain II and domain IV of HER2 ECD.

Mab edit refers to the targeted and precise modification and engineering of antibodies, including the knockout, substitution and addition of sugar in the sugar chains of antibodies, the site-directed or non-site-directed conjugation of antibody payloads, precise improvements in antibody Fab and Fc engineering, and the like. Mab edit focuses on precise modification and engineering, making full use of and regulating various functional regions and different modules of antibodies. By editing native or engineered antibody molecules, novel multi-functional antibodies of various molecular structures, such as glycoengineered antibodies, ADCs, and the like, can be constructed.

Antibody-drug conjugates (ADCs), commonly known as "biological missiles", are novel anti-tumor drugs formed by conjugating a targeting antibody to a drug with strong cytotoxicity. ADCs specifically bind to antigen-positive tumor cell membranes through the targeting of the antibody, playing a pharmacological role. Alternatively, ADCs form endosomes through the endocytosis of tumor cells and enter cells, and in the cytoplasm, endosomes bind to lysosomes, so that the conjugated small-molecule toxin dissociates and recovers its inherent properties, thereby killing tumor cells.

ADCs can be regarded as prodrugs that targetedly deliver small-molecule toxins to tumor cells, increasing the exposure of small-molecule toxins within tumor cells, reducing exposure to normal tissues, and expanding the safety window (therapeutic window) for such small-molecule toxins.

ADCs consist of three major components, namely a targeting antibody, a conjugated toxin, and a linker between the antibody and toxin. Their targets are generally related antigens or specific receptors on tumor cell surfaces. The mAb in an ADC should have the following characteristics: (1) retaining its properties after conjugation to the small-molecule toxin; (2) well targeting the antigen; (3) binding to the antigen on target cells only; (4) not geting negative feedback after binding to the antigen; and (5) barely binding non-specifically to other cells.

The trastuzumab-emtansine conjugate TDM1 is an antibody-drug conjugate obtained by linking the small-molecule toxin DM1 and trastuzumab. In February 2013, TDM1 (Kadcyla) was approved by the FDA as a treatment for HER2-positive advanced metastatic breast cancer. In 2019, TDM1 became the first ADC drug to enter China.

Bispecific antibody-drug conjugates (ADCs) are capable of targeting two different targets, or two different domains of the same target, simultaneously, with promising therapeutic effects. However, there remains a significant unmet clinical need for ADCs using bispecific antibodies.

### SUMMARY

The present invention provides a bispecific antibody-drug conjugate (ADC), a pharmaceutically acceptable salt thereof, a solvate thereof or a deuterated form thereof, comprising a bispecific antibody, a toxin and a linker;
the bispecific antibody comprises a first paratope capable of recognizing and/or binding to domain II of HER2, and a second paratope capable of recognizing and/or binding to domain IV of HER2;
the toxin is selected from the group consisting of maytansines, hemiasterlins, amanitins, auristatins, calicheamicins and duocarmycins;
the linker is a cleavable linker or a non-cleavable linker;
alternatively, one or more hydrogen atoms in the toxin and/or linker are optionally deuterated.

Preferably, the deuterated form means that one or more hydrogen atoms in the toxin and/or linker of the bispecific antibody-drug conjugate (ADC) are optionally deuterated.

In some embodiments, the bispecific antibody comprises a first paratope capable of recognizing and/or binding to domain II of HER2, and a second paratope capable of recognizing and/or binding to domain IV of HER2.

In some embodiments, the bispecific antibody comprises a pair of homologous light chains and a pair of heterologous heavy chains comprising two different heavy chain variable domains.

The pair of homologous light chains comprise a modified trastuzumab light chain variable domain;
the modified trastuzumab light chain variable domain is based on an unmodified trastuzumab light chain variable domain and comprises the following sequence modification(s):
a) any one of S56Y/T/A;
b) any one of N30S/A;
c) any one of T94W/F/Y;
d) any one of H91Y/F/W;
e) any one of P96Y/F/W; or
f) any combination of five kinds of modifications of a) to e);
   in some embodiments, the modified trastuzumab light chain variable domain is based on an unmodified trastuzumab light chain variable domain and comprises the following sequence modification(s): any one, two or three of N30S, S56Y and T94W;
   the sequence of the unmodified trastuzumab light chain variable domain is set forth in SEQ ID NO: 5.

Of the two different heavy chain variable domains,
(1) one is an unmodified pertuzumab heavy chain variable domain or a modified pertuzumab heavy chain variable domain, and the sequence of the unmodified pertuzumab heavy chain variable domain is set forth in SEQ ID NO: 3;
   the modified pertuzumab heavy chain variable domain is based on the unmodified pertuzumab heavy chain variable domain and comprises the following sequence modification(s):
   i) any one of T30A/S/N/D;
   ii) any one of G56A/S/T; or
   iii) any combination of two kinds of modifications of i) and ii);
   in some embodiments, the modified pertuzumab heavy chain variable domain is based on the unmodified pertuzumab heavy chain variable domain and comprises the following sequence modification(s): T30A and/or G56A;
(2) the other heavy chain variable domain is an unmodified trastuzumab heavy chain variable domain or a modified trastuzumab heavy chain variable domain, and the sequence of the unmodified trastuzumab heavy chain variable domain is set forth in SEQ ID NO: 7;
   the modified trastuzumab heavy chain variable domain is based on the unmodified trastuzumab heavy chain variable domain and comprises the following sequence modification(s):
   i) any one of N54T/S/A;
   ii) any one of D98S/W/T/R; or
   iii) any combination of two kinds of modifications of i) and ii);
   in some embodiments, the modified trastuzumab heavy chain variable domain is based on the unmodified trastuzumab heavy chain variable domain and comprises the following sequence modification(s): N54T and/or D98S.

In some embodiments, the bispecific antibody comprises a pair of homologous light chains and a pair of heterologous heavy chains comprising two different heavy chain variable domains;
the pair of homologous light chains comprise a modified trastuzumab light chain variable domain;
the modified trastuzumab light chain variable domain is based on an unmodified trastuzumab light chain variable domain and comprises the following sequence modification(s): any one, two or three of three kinds of modifications ofN30S, S56Y and T94W;
the sequence of the unmodified trastuzumab light chain variable domain is set forth in SEQ ID NO: 5;
of the two different heavy chain variable domains,
   (1) one is an unmodified pertuzumab heavy chain variable domain or a modified pertuzumab heavy chain variable domain, and the sequence of the unmodified pertuzumab heavy chain variable domain is set forth in SEQ ID NO: 3;
      the modified pertuzumab heavy chain variable domain is based on the unmodified pertuzumab heavy chain variable domain and comprises the following sequence modification(s): T30A and/or G56A;
   (2) the other heavy chain variable domain is an unmodified trastuzumab heavy chain variable domain or a modified trastuzumab heavy chain variable domain, and the sequence of the unmodified trastuzumab heavy chain variable domain is set forth in SEQ ID NO: 7;
the modified trastuzumab heavy chain variable domain is based on the unmodified trastuzumab heavy chain variable domain and comprises the following sequence modification(s): N54T and/or D98S.

In some embodiments, the bispecific antibody is in a Fab-Ig, Ig-Fab or heterodimeric Ig format.

In some embodiments, the bispecific antibody is in a heterodimeric Ig format.

In some embodiments, the Fc regions in the bispecific antibody have a knob-into-hole structure, which comprises two complementary Fc regions, and the two complementary Fc regions are an Fc region of the knob part and an Fc region of the hole part.

In some embodiments, the two Fc regions of the bispecific antibody are modified Fc regions that comprise a substitution consisting of M428L.

In some embodiments, the Fc regions of the bispecific antibody include:
(1) an Fc that increases antibody-dependent cellular cytotoxicity (ADCC) effects, or
(2) an Fc that increases antibody-dependent cellular phagocytosis (ADCP), or
(3) an Fc that increases complement-dependent cytotoxicity (CDC) activity.

In some embodiments, the bispecific antibody is a defucosylated antibody.

In some embodiments, the defucosylated antibody is produced by a host cell with a fucosylation deficiency.

In some embodiments, the fucosylation deficiency is from a knockout of the FUT8 gene.

In some embodiments, the defucosylated antibody is obtained by having S239D, I332E or A330L substitutions (Kabat numbering) or a combination of any or all of these mutations in the Fc domain of the antibody.

In some embodiments, the bispecific antibody is in a heterodimeric Ig format in which Fc regions of the two heterologous heavy chains are linked by a knob-into-hole structure;
in some embodiments, the bispecific antibody is in a heterodimeric Ig format in which Fc regions of the two heterologous heavy chains are linked by a knob-into-hole structure, and in which the Fc region of the knob part is an unmodified pertuzumab heavy chain Fc region or comprises the following modification: T366W; the Fc region of the hole part is an unmodified trastuzumab heavy chain Fc region or comprises any combination of the following modifications: T366S, L368A and Y407V;
in some embodiments, the bispecific antibody is in a heterodimeric Ig format in which Fc regions of the two heterologous heavy chains are linked by a knob-into-hole structure, and in which the Fc region of the knob part is based on an unmodified pertuzumab heavy chain Fc and comprises the following sequence modifications: T366W and M428L; the Fc region of the hole part is based on an unmodified trastuzumab heavy chain Fc region and comprises any combination of the following sequence modifications: T366S, L368A and Y407V; and the bispecific antibody further comprises in the Fc region of the hole part the following modification: M428L.

In some embodiments, the bispecific antibody comprises a pair of homologous light chains and a pair of heterologous heavy chains comprising two different heavy chain variable domains;
the pair of homologous light chains comprise a modified trastuzumab light chain variable domain;
the modified trastuzumab light chain variable domain is based on an unmodified trastuzumab light chain variable domain and comprises the following sequence modification(s): any one, two or three of three kinds of modifications ofN30S, S56Y and T94W;
the sequence of the unmodified trastuzumab light chain variable domain is set forth in SEQ ID NO: 5;
of the two different heavy chain variable domains,
   (1) one is an unmodified pertuzumab heavy chain variable domain or a modified pertuzumab heavy chain variable domain, and the sequence of the unmodified pertuzumab heavy chain variable domain is set forth in SEQ ID NO: 3;
      the modified pertuzumab heavy chain variable domain is based on the unmodified pertuzumab heavy chain variable domain and comprises the following sequence modification(s): T30A and/or G56A;
   (2) the other heavy chain variable domain is an unmodified trastuzumab heavy chain variable domain or a modified trastuzumab heavy chain variable domain, and the sequence of the unmodified trastuzumab heavy chain variable domain is set forth in SEQ ID NO: 7;
the modified trastuzumab heavy chain variable domain is based on the unmodified trastuzumab heavy chain variable domain and comprises the following sequence modification(s): N54T and/or D98S;
the bispecific antibody is in a heterodimeric Ig format in which Fc regions of the two heterologous heavy chains are linked by a knob-into-hole structure, and in which the Fc region of the knob part is based on the unmodified pertuzumab heavy chain Fc and comprises the following sequence modifications: T366W and M428L; the Fc region of the hole part is based on the unmodified trastuzumab heavy chain Fc region and comprises any combination of the following sequence modifications: T366S, L368A and Y407V; and the bispecific antibody further comprises in the Fc region of the hole part the following modification: M428L.

In some embodiments, the bispecific antibody comprises a pair of homologous light chains and a pair of heterologous heavy chains comprising two different heavy chain variable domains;
the pair of homologous light chains comprise a modified trastuzumab light chain variable domain;
the modified trastuzumab light chain variable domain is based on an unmodified trastuzumab light chain variable domain and comprises the following sequence modification(s): any one, two or three of three kinds of modifications ofN30S, S56Y and T94W;
the sequence of the unmodified trastuzumab light chain variable domain is set forth in SEQ ID NO: 5;
of the two different heavy chain variable domains,
   (1) one is an unmodified pertuzumab heavy chain variable domain or a modified pertuzumab heavy chain variable domain, and the sequence of the unmodified pertuzumab heavy chain variable domain is set forth in SEQ ID NO: 3;
      the modified pertuzumab heavy chain variable domain is based on the unmodified pertuzumab heavy chain variable domain and comprises the following sequence modification(s): T30A and/or G56A;
   (2) the other heavy chain variable domain is an unmodified trastuzumab heavy chain variable domain or a modified trastuzumab heavy chain variable domain, and the sequence of the unmodified trastuzumab heavy chain variable domain is set forth in SEQ ID NO: 7;
the modified trastuzumab heavy chain variable domain is based on the unmodified trastuzumab heavy chain variable domain and comprises the following sequence modification(s): N54T and/or D98S;
the bispecific antibody is in a heterodimeric Ig format in which Fc regions of the two heterologous heavy chains are linked by a knob-into-hole structure, and in which the Fc region of the knob part is based on the unmodified pertuzumab heavy chain Fc and comprises the following sequence modifications: T366W and M428L; the Fc region of the hole part is based on the unmodified trastuzumab heavy chain Fc region and comprises any combination of the following sequence modifications: T366S, L368A and Y407V; and the bispecific antibody further comprises in the Fc region of the hole part the following modification: M428L;
and the bispecific antibody is a defucosylated antibody.

In some embodiments, the bispecific antibody is selected from the group consisting of T51, T52, T53, T54, T55, T56, T57 and T58.

In some embodiments, the bispecific antibody is selected from the group consisting of defucosylated T51 (T51-AF), defucosylated T52 (T52-AF), defucosylated T53 (T53-AF), defucosylated T54 (T54-AF), defucosylated T55 (T55-AF), defucosylated T56 (T56-AF), defucosylated T57 (T57-AF) and defucosylated T58 (T58-AF).

The toxin is selected from the group consisting of maytansines, hemiasterlins, amanitins, auristatins, calicheamicins or duocarmycins, and deuterated forms thereof.

In some embodiments, the toxin is selected from the group consisting of DM1, DM4, E7974, HTI-286, α-amanitin, β-amanitin, γ-amanitin, ε-amanitin, amanullin, amanin, amaninamide, γ-amanin, monomethyl auristatin F, monomethyl auristatin E, auristatin EB, auristatin EVB, auristatin F phenylenediamine, calicheamicin γ, duocarmycin A, adozelesin and CC-1065, and deuterated forms thereof.

The linker is a cleavable linker or a non-cleavable linker, one or more hydrogen atoms in the linker being optionally deuterated; the cleavable linker includes, but is not limited to: a chemically cleavable linker and/or an enzymatically cleavable linker; examples of the enzymatically cleavable linker include a linker comprising a peptide component.

In some embodiments, the enzymatically cleavable linker is selected from the group consisting of valine-citrulline, phenylalanine-lysine, and deuterated forms thereof.

In some embodiments, the present invention provides a bispecific antibody-drug conjugate (ADC), a pharmaceutically acceptable salt thereof, a solvate thereof or a deuterated form thereof, comprising a bispecific antibody, a toxin and a linker;
the bispecific antibody comprises a pair of homologous light chains and a pair of heterologous heavy chains comprising two different heavy chain variable domains;
the pair of homologous light chains comprise a modified trastuzumab light chain variable domain;
the modified trastuzumab light chain variable domain is based on an unmodified trastuzumab light chain variable domain and comprises the following sequence modification(s): any one, two or three of three kinds of modifications ofN30S, S56Y and T94W;
the sequence of the unmodified trastuzumab light chain variable domain is set forth in SEQ ID NO: 5;
of the two different heavy chain variable domains,
   (1) one is an unmodified pertuzumab heavy chain variable domain or a modified pertuzumab heavy chain variable domain, and the sequence of the unmodified pertuzumab heavy chain variable domain is set forth in SEQ ID NO: 3;
      the modified pertuzumab heavy chain variable domain is based on the unmodified pertuzumab heavy chain variable domain and comprises the following sequence modification(s): T30A and/or G56A;
   (2) the other heavy chain variable domain is an unmodified trastuzumab heavy chain variable domain or a modified trastuzumab heavy chain variable domain, and the sequence of the unmodified trastuzumab heavy chain variable domain is set forth in SEQ ID NO: 7;
the modified trastuzumab heavy chain variable domain is based on the unmodified trastuzumab heavy chain variable domain and comprises the following sequence modification(s): N54T and/or D98S;
the bispecific antibody is in a heterodimeric Ig format in which Fc regions of the two heterologous heavy chains are linked by a knob-into-hole structure, and in which the Fc region of the knob part is based on the unmodified pertuzumab heavy chain Fc and comprises the following sequence modifications: T366W and M428L; the Fc region of the hole part is based on the unmodified trastuzumab heavy chain Fc region and comprises any combination of the following sequence modifications: T366S, L368A and Y407V; and the bispecific antibody further comprises in the Fc region of the hole part the following modification: M428L;
the bispecific antibody is a defucosylated antibody;
the toxin is selected from the group consisting of DM1, DM4, E7974, HTI-286, monomethyl auristatin F, monomethyl auristatin E, auristatin EB, auristatin EVB, auristatin F phenylenediamine, and deuterated forms thereof;
the linker is selected from the group consisting of valine-citrulline, phenylalanine-lysine, and deuterated forms thereof.

In some embodiments, the present invention provides a bispecific antibody-drug conjugate (ADC), a pharmaceutically acceptable salt thereof, a solvate thereof or a deuterated form thereof, comprising a bispecific antibody, a toxin and a linker;
the bispecific antibody comprises a pair of homologous light chains and a pair of heterologous heavy chains comprising two different heavy chain variable domains;
the pair of homologous light chains comprise a modified trastuzumab light chain variable domain;
the modified trastuzumab light chain variable domain is based on an unmodified trastuzumab light chain variable domain and comprises the following sequence modification(s): any one, two or three of three kinds of modifications ofN30S, S56Y and T94W;
the sequence of the unmodified trastuzumab light chain variable domain is set forth in SEQ ID NO: 5;
of the two different heavy chain variable domains,
   (1) one is an unmodified pertuzumab heavy chain variable domain or a modified pertuzumab heavy chain variable domain, and the sequence of the unmodified pertuzumab heavy chain variable domain is set forth in SEQ ID NO: 3;
      the modified pertuzumab heavy chain variable domain is based on the unmodified pertuzumab heavy chain variable domain and comprises the following sequence modification(s): T30A and/or G56A;
   (2) the other heavy chain variable domain is an unmodified trastuzumab heavy chain variable domain or a modified trastuzumab heavy chain variable domain, and the sequence of the unmodified trastuzumab heavy chain variable domain is set forth in SEQ ID NO: 7;
the modified trastuzumab heavy chain variable domain is based on the unmodified trastuzumab heavy chain variable domain and comprises the following sequence modification(s): N54T and/or D98S;
the bispecific antibody is in a heterodimeric Ig format in which Fc regions of the two heterologous heavy chains are linked by a knob-into-hole structure, in which the Fc region of the knob part is based on the unmodified pertuzumab heavy chain Fc and comprises the following sequence modifications: T366W and M428L; the Fc region of the hole part is based on the unmodified trastuzumab heavy chain Fc region and comprises any combination of the following sequence modifications: T366S, L368A and Y407V; and the bispecific antibody further comprises in the Fc region of the hole part the following modification: M428L;
the bispecific antibody is a defucosylated antibody;
the toxin is selected from the group consisting of monomethyl auristatin E and deuterated forms thereof;
the linker is selected from the group consisting of valine-citrulline and deuterated forms thereof.

In some embodiments, the present invention provides a bispecific antibody-drug conjugate (ADC), a pharmaceutically acceptable salt thereof, a solvate thereof or a deuterated form thereof, comprising a bispecific antibody, a toxin and a linker;
(1) the bispecific antibody is selected from the group consisting of T51, T52, T53, T54, T55, T56, T57 and T58; in some embodiments, the bispecific antibody is a defucosylated antibody: T51-AF, T52-AF, T53-AF, T54-AF, T55-AF, T56-AF, T57-AF or T58-AF;
(2) the toxin is selected from the group consisting of DM1, DM4, E7974, HTI-286, monomethyl auristatin F, monomethyl auristatin E, auristatin EB, auristatin EVB, auristatin F phenylenediamine, and deuterated forms thereof; in some embodiments, the toxin is selected from the group consisting of monomethyl auristatin E and deuterated forms thereof;
(3) the linker is selected from the group consisting of valine-citrulline, phenylalanine-lysine, and deuterated forms thereof; in some embodiments, the linker is valine-citrulline or a deuterated form thereof.

In some embodiments, the present invention provides a bispecific antibody-drug conjugate (ADC), a pharmaceutically acceptable salt thereof, a solvate thereof or a deuterated form thereof, comprising a bispecific antibody, a toxin and a linker;
(1) the bispecific antibody is selected from the group consisting of T51, T52, T53, T54, T55, T56, T57 and T58, and the defucosylated antibodies T51-AF, T52-AF, T53-AF, T54-AF, T55-AF, T56-AF, T57-AF and T58-AF;
(2) the toxin is selected from the group consisting of DM1, DM4, E7974, HTI-286, monomethyl auristatin F, monomethyl auristatin E, auristatin EB, auristatin EVB, auristatin F phenylenediamine, and deuterated forms thereof;
(3) the linker is selected from the group consisting of valine-citrulline, phenylalanine-lysine, and deuterated forms thereof.

In some embodiments, the present invention provides a bispecific antibody-drug conjugate (ADC), a pharmaceutically acceptable salt thereof, a solvate thereof or a deuterated form thereof, comprising a bispecific antibody, a toxin and a linker;
(1) the bispecific antibody is selected from the group consisting of T51, T52, T53, T54, T55, T56, T57 and T58, and the defucosylated antibodies T51-AF, T52-AF, T53-AF, T54-AF, T55-AF, T56-AF, T57-AF and T58-AF;
(2) the toxin is selected from the group consisting of monomethyl auristatin E and deuterated forms thereof;
(3) the linker is selected from the group consisting of valine-citrulline and deuterated forms thereof.

In some embodiments, the valine-citrulline linker is linked to the bispecific antibody by 6-maleimidohexanoic acid and to monomethyl auristatin E toxin by p-aminobenzyl alcohol.

In some embodiments, the average drug-to-antibody ratio (DAR) of the bispecific antibody-drug conjugate is about 1-6, e.g., about 1, 2, 3, 4, 5 or 6, e.g., about 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9 or 5.

In some embodiments, the structure of the bispecific antibody-drug conjugate is represented by Formula (I): 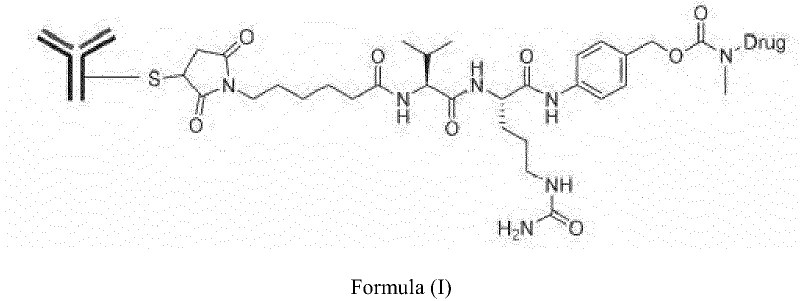 wherein 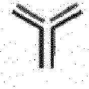 represents the bispecific antibody described above, and the middle part is the linker moiety, valine-citrulline (Val-Cit, abbreviated as Vc); the linker is linked to the bispecific antibody by 6-maleimidohexanoic acid and to the rightmost Drug (the toxin moiety) by p-aminobenzyl alcohol.

In some embodiments, the DAR value (n) is further indicated in the structure of the bispecific antibody-drug conjugate as shown in Formula (I'): 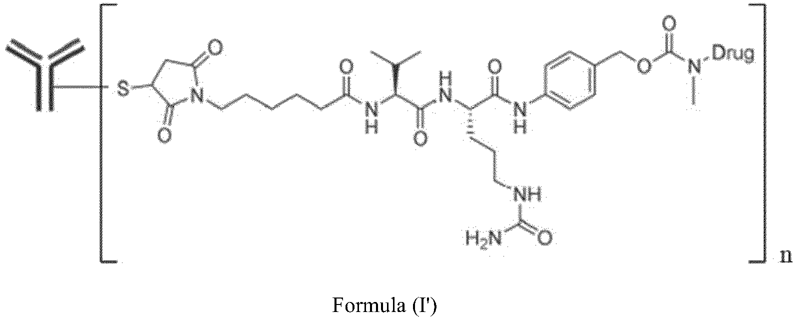 wherein 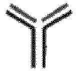 represents the bispecific antibody described above, and the middle part is the linker moiety, valine-citrulline (Val-Cit, abbreviated as Vc); the linker is linked to the bispecific antibody by 6-maleimidohexanoic acid and to the rightmost Drug (the toxin moiety) by p-aminobenzyl alcohol; the toxin is selected from the group consisting of DM1, DM4, E7974, HTI-286, monomethyl auristatin F, monomethyl auristatin E, auristatin EB, auristatin EVB, auristatin F phenylenediamine, and deuterated forms thereof;
n represents the average drug-to-antibody ratio (DAR), and n is selected from 1-6, e.g., about 1, 2, 3, 4, 5 or 6, e.g., about 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9 or 5.

In some embodiments, the structure of the bispecific antibody-drug conjugate is represented by Formula (I'): 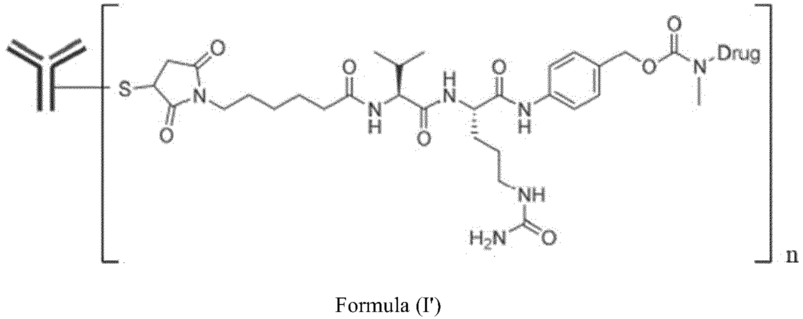 wherein 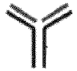 represents a bispecific antibody selected from the group consisting of T51, T52, T53, T54, T55, T56, T57 and T58, and the defucosylated antibodies T51-AF, T52-AF, T53-AF, T54-AF, T55-AF, T56-AF, T57-AF and T58-AF; the middle part is the linker moiety, valine-citrulline (Val-Cit, abbreviated as Vc); the linker is linked to the bispecific antibody by 6-maleimidohexanoic acid and to the rightmost Drug (the toxin moiety) by p-aminobenzyl alcohol; the toxin is selected from the group consisting of DM1, DM4, E7974, HTI-286, monomethyl auristatin F, monomethyl auristatin E, auristatin EB, auristatin EVB, auristatin F phenylenediamine, and deuterated forms thereof;
n represents the average drug-to-antibody ratio (DAR), and n is selected from 3-4, e.g., about 3, 3.1, 3.2, 3.3, 3.4, 3.5, 3.6, 3.7, 3.8, 3.9 or 4.

In some embodiments, the structure of the bispecific antibody-drug conjugate is represented by Formula (II): 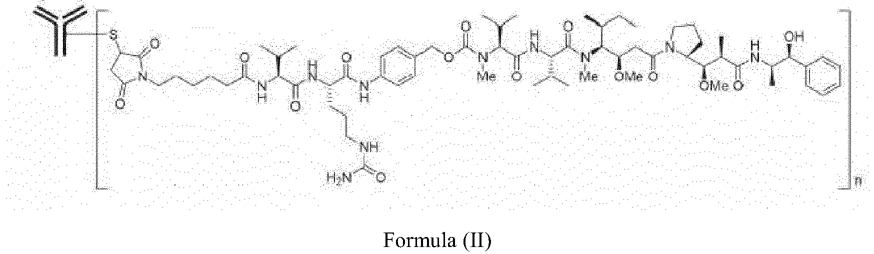 wherein 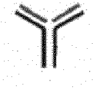 represents the bispecific antibody described above, and the bracketed structure is the linker-toxin moiety, Vc-MMAE; Vc is linked to the bispecific antibody by 6-maleimidohexanoic acid and to the toxin monomethyl auristatin E (MMAE) by p-aminobenzyl alcohol. In the formula, n is the DAR value, and n is 1-6, e.g., about 1, 2, 3, 4, 5 or 6, e.g., 3-4, e.g., about 2.5, 2.6, 2.7, 2.8, 2.9, 3, 3.1, 3.2, 3.3, 3.4, 3.5, 3.6, 3.65, 3.7, 3.75, 3.8, 3.9, 4, 4.1, 4.2, 4.3, 4.4, 4.5, 4.6, 4.7, 4.8, 4.9 or 5.

In some embodiments, the average drug-to-antibody ratio (DAR) of the bispecific antibody-drug conjugate is 3-4, e.g., 3, 3.1, 3.2, 3.3, 3.4, 3.5, 3.6, 3.65, 3.7, 3.75, 3.8, 3.9 or 4.

In some embodiments, the structure of the bispecific antibody-drug conjugate is represented by Formula (II): 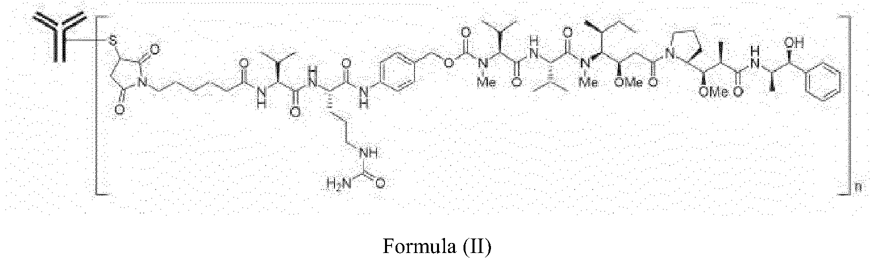 wherein 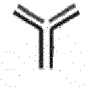 represents a bispecific antibody selected from the group consisiting of T51, T52, T53, T54, T55, T56, T57 and T58, and the defucosylated antibodies T51-AF, T52-AF, T53-AF, T54-AF, T55-AF, T56-AF, T57-AF and T58-AF; the bracketed structure is the linker-toxin moiety, Vc-MMAE; Vc is linked to the bispecific antibody by 6-maleimidohexanoic acid and to the toxin monomethyl auristatin E (MMAE) by p-aminobenzyl alcohol. In the formula, n is the DAR value, and n is selected from 3-4, e.g., about 3, 3.1, 3.2, 3.3, 3.4, 3.5, 3.6, 3.7, 3.8, 3.9 or 4.

In some embodiments, the structure of the bispecific antibody-drug conjugate is represented by Formula (II): 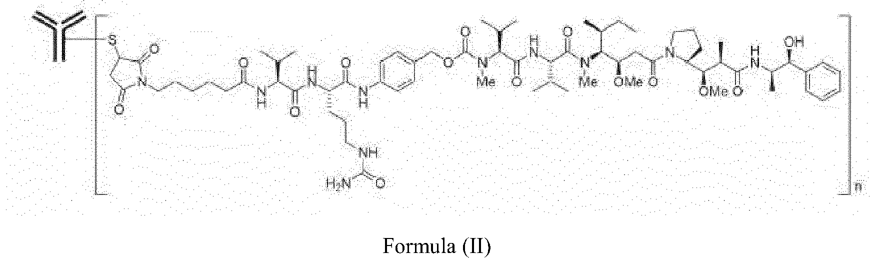 wherein 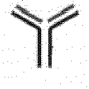 represents a bispecific antibody selected from the group consisting of T54, T58, and the defucosylated antibodies T54-AF and T58-AF; the bracketed structure is the linker-toxin moiety, Vc-MMAE; Vc is linked to the bispecific antibody by 6-maleimidohexanoic acid and to the toxin monomethyl auristatin E (MMAE) by p-aminobenzyl alcohol. In the formula, n is the DAR value, and n is selected from 3-4, e.g., about 3, 3.1, 3.2, 3.3, 3.4, 3.5, 3.6, 3.7, 3.8, 3.9 or 4.

In some embodiments, the bispecific antibody is selected from the group consisting of T51, T52, T53, T54, T55, T56, T57 and T58.

In some embodiments, the bispecific antibody is selected from the group consisting of defucosylated T51 (T51-AF), defucosylated T52 (T52-AF), defucosylated T53 (T53-AF), defucosylated T54 (T54-AF), defucosylated T55 (T55-AF), defucosylated T56 (T56-AF), defucosylated T57 (T57-AF) and defucosylated T58 (T58-AF).

In some embodiments, the bispecific antibody is selected from the group consisting of T54, T58, defucosylated T54 (T54-AF), and defucosylated T58 (T58-AF).

In some embodiments, the bispecific antibody is defucosylated T54 (T54-AF) or defucosylated T58 (T58-AF).

In some embodiments, the bispecific antibody-drug conjugate of the present invention is T51-MMAE, T52-MMAE, T53-MMAE, T54-MMAE, T55-MMAE, T56-MMAE, T57-MMAE, T58-MMAE, or the corresponding defucosylated bispecific antibody-drug conjugate T51-AF-MMAE, T52-AF-MMAE, T53-AF-MMAE, T54-AF-MMAE, T55-AF-MMAE, T56-AF-MMAE, T57-AF-MMAE or T58-AF-MMAE.

In some embodiments, the bispecific antibody-drug conjugate of the present invention is T54-MMAE or T58-MMAE, or the corresponding defucosylated bispecific antibody-drug conjugate T54-AF-MMAE or T58-AF-MMAE.

According to another aspect of the present invention, provided is a pharmaceutical composition comprising the bispecific antibody-drug conjugate, the pharmaceutically acceptable salt thereof, the solvate thereof or the deuterated form thereof described above, and a pharmaceutically acceptable carrier or excipient.

According to another aspect of the present invention, provided is a use of the bispecific antibody-drug conjugate, the pharmaceutically acceptable salt thereof, the solvate thereof or the deuterated form thereof described above, or the pharmaceutical composition described above, in the preparation of a medicament for preventing and/or treating cancer.

In some embodiments, the cancer is a HER2-expressing cancer.

In some embodiments, the cancer is a cancer with a moderate-to-low level of HER2 expression.

In some embodiments, the cancer includes breast cancer, ovarian cancer, endometrial cancer, cervical cancer, gastric cancer, esophageal cancer, lung cancer, head and neck cancer, bladder cancer, bile duct cancer, and colorectal cancer.

The present invention also relates to a use of the bispecific antibody-drug conjugate, the pharmaceutically acceptable salt thereof, the solvate thereof or the deuterated form thereof or the pharmaceutical composition in the preparation of an anti-tumor combined formulation, and the combined formulation further comprises the following optional active ingredients for treating tumors:
(1) an agent that targets a target other than HER2;
(2) an agent for cell-therapy; and
(3) an agent for immunotherapy.

According to another aspect of the present invention, provided is a method for preventing and/or treating cancer, comprising administering to a patient in need thereof a therapeutically effective amount of the bispecific antibody-drug conjugate, the pharmaceutically acceptable salt thereof, the solvate thereof or the deuterated form thereof described above, or the pharmaceutical composition described above.

In some embodiments, the cancer is a HER2-expressing cancer.

In some embodiments, the cancer is a cancer with a moderate-to-low level of HER2 expression.

In some embodiments, the cancer includes breast cancer, ovarian cancer, endometrial cancer, cervical cancer, gastric cancer, esophageal cancer, lung cancer, head and neck cancer, bladder cancer, bile duct cancer, and colorectal cancer.

The present invention also relates to a use of the bispecific antibody-drug conjugate, the pharmaceutically acceptable salt thereof, the solvate thereof or the deuterated form thereof, or the pharmaceutical composition, as described above, in the treatment of cancer, wherein the bispecific antibody-drug conjugate, the pharmaceutically acceptable salt thereof, the solvate thereof or the deuterated form thereof, or the pharmaceutical composition, is used in combination with the following drugs or treatment regimens for treating cancer:
(1) a drug that targets a target other than HER2;
(2) a cell-therapy drug;
(3) an immunotherapy drug;
(4) a surgery; and
(5) a physical therapy such as radiotherapy.

The present invention has the following beneficial effects:
(1) The bispecific antibody-drug conjugate, the pharmaceutically acceptable salt thereof, the solvate thereof or the deuterated form thereof according to the present invention has excellent anti-tumor activity: it has better anti-tumor activity in multiple mouse tumor models of different types than TDM1 (or DS8201) at specific doses and can better inhibit tumor growth in a shorter time than TDM1 (or DS8201) at identical doses.
(2) The bispecific antibody-drug conjugate, the pharmaceutically acceptable salt thereof, the solvate thereof or the deuterated form thereof according to the present invention can be well tolerated and has low toxicity.

### BRIEF DESCRIPTION OF THE DRAWINGS

FIG. 1 shows a schematic of a bispecific antibody with homologous light chains, in a Fab-Ig format, designed from antibody A and antibody B.
FIG. 2 shows a schematic of a bispecific antibody with homologous light chains, in an Ig-Fab format, designed from antibody A and antibody B.
FIG. 3 shows a schematic of a bispecific antibody with homologous light chains, in a heterodimeric IgG format, designed from antibody A and antibody B, where the heterodimers are linked by a knob-into-hole structure.
FIG. 4 shows a schematic of a bispecific antibody with homologous light chains, in a heterodimeric IgG format, designed from antibody A and antibody B, where the heterodimers are linked by electrostatic steering mechanism.
FIG. 5 shows SEC purity profiles of the antibodies T54 (5A) and T54-AF (5B), where the abscissa represents time (min) and the ordinate represents absorbance (mAU).
FIG. 6 shows SEC purity profiles of the antibody-drug conjugates T54-MMAE (6A) and T54-AF-MMAE (6B), where the abscissa represents time (min) and the ordinate represents absorbance (mAU).
FIG. 7 shows a HIC-HPLC profile of the antibody T54, where the abscissa represents time (min) and the ordinate represents absorbance (mAU).
FIG. 8 shows HIC-HPLC profiles of the antibody-drug conjugates T54-MMAE (8A) and T54-AF-MMAE (8B), where the abscissa represents time (min) and the ordinate represents absorbance (mAU).
FIG. 9 shows the binding curves of a positive control ADC (DS8201), T54-AF and T54-AF-MMAE (T54-AF-ADC) to the HER2 antigen, where the abscissa represents the logarithm of concentration and the ordinate represents the OD₄₅₀ value.
FIG. 10 shows the inhibition curves of the antibodies Herceptin and T54 and their respective conjugates TDM1 and T54-MMAE on the growth of the breast cancer cell SKBR3, where the abscissa represents the logarithm of concentration and the ordinate represents the cell viability of the breast cancer cell SKBR3.
FIG. 11 shows the inhibition curves of the antibodies Herceptin and T54 and their respective conjugates TDM1 and T54-MMAE on the growth of the gastric cancer cell N87, where the abscissa represents the logarithm of concentration and the ordinate represents the cell viability of the gastric cancer cell N87.
FIG. 12 shows the inhibitory effects of T54-AF-MMAE, DS8201, T54-AF, Herceptin, Perjeta, Isotype (isotype control, as a negative antibody) on different cell strains, where (A) the BT474 cell strain, (B) the NCI-H2170 cell strain, (C) the MDA-MB-175VII cell strain, (D) the SKOV-3 cell strain, (E) the MCF-7 cell strain, (F) the MDA-MB-435 cell strain, and (G) the NCI-H446 cell strain.
FIG. 13 shows growth curves of the human xenograft breast cancer model BT474 upon the treatment with TDM1 and the treatment with T54-MMAE antibody-drug conjugate, where the abscissa represents days after tumor inoculation and the ordinate represents tumor volume (mm³).
FIG. 14 shows growth curves of the human xenograft breast cancer model BT474 upon the treatment with a positive control ADC (DS8201) and the treatment with the defucosylated bispecific antibody-drug conjugate T54-AF-MMAE (T54-AF-ADC), where the abscissa represents days after tumor inoculation and the ordinate represents tumor volume (mm³).
FIG. 15 shows growth curves of the human xenograft gastric cancer model N87 upon the treatment with a positive control ADC (DS8201) and the treatment with the defucosylated bispecific antibody-drug conjugate T54-AF-MMAE (T54-AF-ADC), where the abscissa represents days after tumor inoculation and the ordinate represents tumor volume (mm³).

### DETAILED DESCRIPTION

The present invention discloses a bispecific antibody-drug conjugate, a pharmaceutically acceptable salt thereof, a solvate thereof or a deuterated form thereof, comprising a bispecific antibody, a toxin and a linker; the bispecific antibody recognizes and/or binds to domain II of HER2 and recognizes and/or binds to domain IV of HER2; the toxin is selected from the group consisting of maytansines, hemiasterlins, amanitins, auristatins, calicheamicins or duocarmycins; the linker is a cleavable linker or a non-cleavable linker; one or more hydrogen atoms in the toxin and linker are optionally deuterated.

As used herein, the term "antibody" refers to a molecule having the general structure of a naturally occurring mammalian immunoglobulin (Ig) (IgG is a paradigmatic example thereof). That is, the antibody comprises two identical light chains comprising one variable domain and one constant domain, and two identical heavy chains comprising one variable domain and three constant domains. A light chain and a heavy chain associate with each other through their variable domains, as well as the constant domain of the light chain and the first constant domain of the heavy chain. The two heavy chains associate with each other through the 2^{nd} and 3^{rd} constant domains. The antigen-binding site of the antibody is formed from two variable domains and particularly from the three complementarity determining regions (CDRs) of each variable domain. An antibody is said to bind a molecule (an antigen) if it is capable of specifically interacting with and adhering to the molecule. Antibody binding does not include non-specific or low-affinity interactions. Although "antibody" is intended to mean the structure of naturally occurring immunoglobulins, it also includes designed molecules retaining this general structure, such as chimeric, CDR-grafted and humanized antibodies.

As used herein, the term "epitope" refers to a part of an antigen that mediates specific binding of the antigen to an antibody (or antibody construct) by contacting with the antigen-binding site of the antibody (or antibody construct).

As used herein, the term "paratope" refers to a part of an antibody (or antibody construct) that mediates specific binding of the antibody (or antibody construct) to an antigen by contacting with the epitope of the antigen.

As used herein, the terms "modification", "mutation" and "substitution" (and grammatical forms thereof) refer to engineered changes in amino acid sequences. Unless context dictates otherwise, a mutation refers to a sequence change arising from a human-assisted biological process. Conventionally, a substitution is denoted by an amino acid in single-letter code from the reference sequence, its position in the reference sequence, and an amino acid in single-letter code from the resultant sequence. For example, A26S donates that alanine (A) at position 26 in the reference sequence is changed to serine (S) in the resultant sequence.

As used herein, the term "homologous" means that the amino acid sequences of two or more peptide chains are completely identical.

As used herein, the term "heterologous" means that there is difference between the amino acid sequences of two or more peptide chains, i.e., they are not completely identical.

As used herein, the terms "deuterated form" and "deuterated" refer to a structure resulting from the substitution of one or more hydrogen atoms (H-1) in an antibody-drug conjugate structure with deuterium atoms (H-2).

Any atom of a compound in the present invention may represent any stable isotope of this atom, if not specified. Unless otherwise specified, when a position in a structure is defined as H, i.e., hydrogen (H-1), this position contains only the naturally occurring isotope. Likewise, when a position in a structure is defined as D, i.e., deuterium (H-2), unless otherwise specified, the amount of the isotope at this position is at least 3340 times greater than that of the naturally occurring isotope (0.015%) (i.e., at least 50.1% deuterium isotope is contained). When one or several positions in the structure of a compound of the present application are defined as D, i.e., deuterium (H-2), the content of the compound represented by the structure can be at least 52.5%, at least 60%, at least 67.5%, at least 75%, at least 82.5%, at least 90%, at least 95%, at least 97%, at least 98.5%, at least 99%, or at least 99.5%.

The deuteration ratio of a compound of the present application refers to the ratio of the content of a labeled synthetic isotope to the content of the naturally occurring isotope. The deuteration ratio of each specified deuterium atom of a compound of the present application can be at least 3500 times (52.5%), at least 4000 times (60%), at least 4500 times (67.5%), at least 5000 times (75%), at least 5500 times (82.5%), at least 6000 times (90%), at least 6333.3 times (95%), at least 6466.7 times (97%), at least 6566.7 times (98.5%), at least 6600 times (99%), or at least 6633.3 times (99.5%).

Isotopologues in the present application refer to compounds that differ only in their isotopic composition in terms of chemical structure. A compound containing deuterium at a specific position in the present application also contains a tiny amount of hydrogen isotopologues at that position. The amount of hydrogen isotopologues at a deuterated position in a deuterated compound of the present application depends on many factors, including the purity of the deuterium isotope of the deuterated agent (D₂O, D₂, NaBD₄, LiAlD₄, etc.) and the effectiveness of the synthesis method that introduces the deuterium isotope. However, as previously mentioned, the total amount of hydrogen isotopologues at such a deuterated position will be less than 49.9%. The total amount of hydrogen isotopologues at a deuterated position in a deuterated compound of the present application will be less than 47.5%, 40%, 32.5%, 25%, 17.5%, 10%, 5%, 3%, 1% or 0.5%.

In the present application, any atom not designated as deuterium is present in its natural isotopic abundance.

In the present application, numbering for the amino acid sequences of light chain variable domains, as well as the amino acid sequences of heavy chain variable domains, is based on Vajdos et al., J. Mol. Biol. 320:415 (2002) and uses the Kabat numbering scheme (Kabat *et al.*, NIH publication no. 91-3242, pp 662,680,689 (1991).

### Bispecific Antibody

The bispecific antibody of the present invention can have many different general structures. In some embodiments, the bispecific antibody of the present invention is specific for two epitopes in the same antigen or different antigens. In some embodiments, the bispecific antibody of the present invention has two paratopes.

It will be appreciated that additional variable domains may be added to each heavy chain such that the heavy chain comprises, for example, 3, 4, 5 or more variable domains that contribute to the formation of an equal number of paratopes. In these multi-paratope antibodies, the third paratope may confer specificity for a third epitope, rendering the antibody trispecific. Alternatively, the third paratope may be specific for one of the epitopes recognized by either of the first two paratopes so that the antibody remains bispecific but has increased valence for one of the epitopes. Similarly, the fourth paratope may be specific for a fourth epitope so that the antibody construct is tetraspecific, or it may be specific for one of the epitopes recognized by any of the first three paratopes so that the antibody construct is trispecific or bispecific.

In some embodiments, the bispecific antibody comprises a first paratope capable of recognizing and/or binding to domain II of HER2, and a second paratope capable of recognizing and/or binding to domain IV of HER2.

In some embodiments, the bispecific antibody comprises a pair of homologous light chains and a pair of heterologous heavy chains comprising two different heavy chain variable domains.

In some embodiments, the pair of homologous light chains comprise a modified trastuzumab light chain variable domain;
the modified trastuzumab light chain variable domain is based on an unmodified trastuzumab light chain variable domain and comprises the following sequence modification(s):
   a) any one of S56Y/T/A;
   b) any one of N30S/A;
   c) any one of T94W/F/Y;
   d) any one of H91Y/F/W;
   e) any one of P96Y/F/W; or
   f) any combination of five kinds of modifications of a) to e);
in some embodiments, the modified trastuzumab light chain variable domain is based on an unmodified trastuzumab light chain variable domain and comprises the following sequence modification(s): any one, two or three of three kinds of modifications of N30S, S56Y and T94W;
the sequence of the unmodified trastuzumab light chain variable domain is set forth in SEQ ID NO: 5.

Of the two different heavy chain variable domains,
(1) one is selected from an unmodified pertuzumab heavy chain variable domain or a modified pertuzumab heavy chain variable domain, and the sequence of the unmodified pertuzumab heavy chain variable domain is set forth in SEQ ID NO: 3;
   the modified pertuzumab heavy chain variable domain is based on the unmodified pertuzumab heavy chain variable domain and comprises the following sequence modification(s):
   i) T30A/S/N/D;
   ii) G56A/S/T; or
   iii) any combination of two kinds of modifications of i) and ii);
      in some embodiments, the modified pertuzumab heavy chain variable domain is based on the unmodified pertuzumab heavy chain variable domain and comprises the following sequence modification(s): T30A and/or G56A;
(2) the other heavy chain variable domain is selected from an unmodified trastuzumab heavy chain variable domain or a modified trastuzumab heavy chain variable domain, and the sequence of the unmodified trastuzumab heavy chain variable domain is set forth in SEQ ID NO: 7;
the modified trastuzumab heavy chain variable domain is based on the unmodified trastuzumab heavy chain variable domain and comprises the following sequence modification(s):
i) N54T/S/A;
ii) D98S/W/T/R; or
iii) any combination of two kinds of modifications of i) and ii);
in some embodiments, the modified trastuzumab heavy chain variable domain is based on the unmodified trastuzumab heavy chain variable domain and comprises the following sequence modification(s): N54T and/or D98S.

In some embodiments, the bispecific antibody is in a Fab-Ig, Ig-Fab or heterodimeric Ig format.

In some embodiments, the bispecific antibody is in a heterodimeric Ig format.

As used herein, the term "Fc region", "Fc" or "Fc domain" refers to a C-terminal region of an immunoglobulin heavy chain that contains at least a portion of a constant region. The term includes native sequence Fc regions and variant Fc regions. Unless otherwise indicated herein, numbering of amino acid residues in an Fc region or constant region is according to the EU numbering system, also known as the EU index, as described in Kabat et al., Sequences of Proteins of Immunological Interest, 5th edition, Public Health Service, National Institutes of Health, Bethesda, MD (1991).

The constant domains (C_{H1}, C_{H2} and C_{H3}) are those of human immunoglobulins such as IgG, IgM, IgA, IgD; or IgG and its isotypes IgG1, IgG2, IgG3 and IgG4; or combinations of C_{H1}, C_{H2} and C_{H3} domains recombined from these types and isotypes.

In some embodiments, the Fc of the bispecific antibody includes an Fc with increased antibody-dependent cellular cytotoxicity (ADCC), antibody-dependent cellular phagocytosis (ADCP) or complement-dependent cytotoxicity (CDC) activity, which results from increase or decrease in binding affinity for Fc receptors (such as CD16a, CD16b, CD32a, CD16b, CD64 and C1q protein). This includes but is not limited to ADCC-enhanced defucosylated antibodies, which are obtained (1) by producing bispecific antibodies in host cells with fucosylation deficiencies, such as from a knockout of the FUT8 gene (Yamane-Ohnuki N et al., Biotechnol Bioeng. 87(5):614-22, 2004); (2) by having S239D, 1332E, A330L substitutions (Kabat numbering) or a combination of any or all of these mutations in the Fc domain of the antibody (Lazar GA et al., Proc Natl Acad Sci U S A. 103(11): 4005-4010, 2006); or (3) as disclosed in the Chinese patent application CN201610194325.7.

In some embodiments, the bispecific antibody comprises mutations in the Fc domain to reduce ADCC activity or CDC activity. These may include, but are not limited to, in the Fc domain, (1) a mutation of N297, such as but not limited to, N297A or N297G; (2) a mutation of L234, such as L234A or L234G, and/or a mutation of L235, such as L235A or L235G; (3) a mutation of P329, such as P329G; or (4) a mutation of D265, such as D265A; or a combination of substitutions at any or all of these positions.

In some embodiments, the Fc mutations (all numberings are based on Eu indexes of the Kabat numbering system) include mutation(s) that increase the serum half-life. In one embodiment, the Fc has the following substitution: T250Q, or M428L, or a T250Q/M428L double mutation in C_{H3} (Hinton et al., J Biol Chem. 279(8):6213-6, 2004). In another embodiment, the Fc of the bispecific antibody has an M252Y/S254T/T256E triple mutation (Dall'Acqua WF et al., J Immunol 169(9):5171-80, 2002). In another embodiment, the Fc of the bispecific antibody has an N434A mutation (Petkova SB et al., International Immunology 18(12): 1759-1769, 2006.) or an M428L/N434S double mutation, or an M428L/N434A double mutation (Zalevsky J et al., Nat Biotechnol. 28(2): 157-159, 2010).

In some embodiments, the Fc regions of the bispecific antibody have been modified to increase its serum half-life. In some embodiments, the modification that increases the serum half-life is M428L.

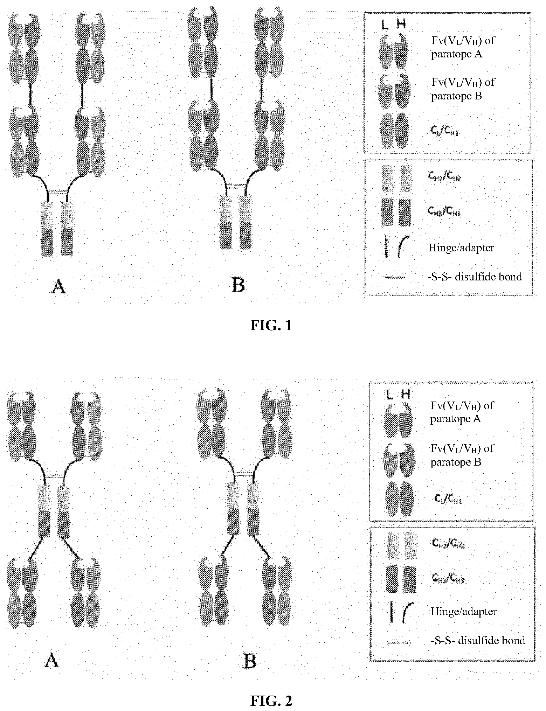

The bispecific antibody of the present invention may be in a homodimeric format (comprising two identical heavy chains), such as Fab-Ig (as shown in FIG. 1) or Ig-Fab (as shown in FIG. 2). The adapter consists of 0 to 100 amino acids of any composition. In some embodiments, the adapter is, for example, (G₃S)ₙG₃, wherein n is any number between 1 and 20 (as shown in Table 8).

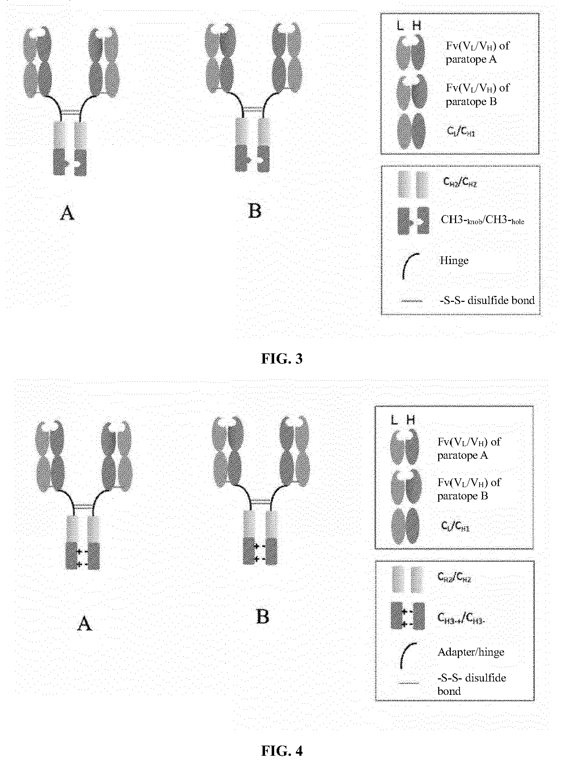

The bispecific antibody of the present invention may also be in a heterodimeric Ig format. Two different heavy chains from two different antibodies can form a heterodimer using technologies described in the art, including but not limited to, knobs-into-holes (as shown in FIG. 3), the electrostatic steering mechanism (as shown in FIG. 4), and the like.

In some embodiments, the bispecific antibody of the present invention is selected from those disclosed in the international patent publication WO2018/191188A1.

In some embodiments, the bispecific antibody of the present invention is selected from the group consisting of T51, T52, T53, T54, T55, T56, T57 and T58.

Among the 8 bispecific antibodies T51 to T58, T54 and T58 are in a heterodimeric Ig format and have a knob-into-hole structure, and the other 6 are in a homodimeric format. Among them, T53 and T57 are in an Ig-Fab format, and T51, T52, T55 and T56 are in a Fab-Ig format.

The heavy chain codes and amino acid sequences of the 8 bispecific antibodies T51-T58 are shown in Table 8 and Table 9.

The homologous light chains of T51 to T58 are based on an unmodified trastuzumab light chain variable domain and comprise, for example, the following site mutation(s): mutation of N30 to S (N30S), mutation of S56 to Y (S56Y) or mutation of T94 to W (T94W), or a combination of any two or three of the above mutations, for example, N30S/S56Y, N30S/T94W, S56Y/T94W, N30S/S56Y/T94W, or the like. The light chain codes corresponding to T51 to T58 are shown in Table 8, and the sequence of the trastuzumab light chain variable domain as basis is set forth in SEQ ID NO: 5.

The bispecific antibody disclosed herein can be produced by a recombination method. The method for recombination production is well known in the art and comprises: protein expression in prokaryotic and eukaryotic cells, and subsequent isolation of bispecific antibodies, and usually purification to a pharmaceutically acceptable purity. For the expression of the above antibody in host cells, a nucleic acid that encodes the antibody sequence is inserted into an expression vector by a standard method. Expression is carried out in suitable prokaryotic or eukaryotic host cells, such as CHO cells, NSO cells, SP2/0 cells, HEK293 cells, COS cells, PER.C6 cells, yeast or *E. coli* cells, and the antibody is recovered from the cells (lysed cells or supernatant).

Thus, certain embodiments disclosed herein include a method for preparing a bispecific antibody, comprising the following steps: a) transforming a host cell with at least one expression vector comprising a nucleic acid molecule encoding the antibody; b) culturing the host cell under conditions that allow synthesis of the antibody molecule; and c) recovering the antibody from the culture.

The antibody is suitably separated from the culture medium by conventional immunoglobulin purification procedures such as protein A-Sepharose, hydroxylapatite chromatography, gel electrophoresis, dialysis or affinity chromatography.

As used herein, the expressions "cell", "cell line" and "cell culture" are used interchangeably and all such designations include their progeny. The words "transformant" and "transformed cell" include the primary test cells and cultures derived therefrom, regardless of the number of passages. It will also be appreciated that all progeny may not be identical in DNA content due to deliberate or inadvertent mutations, and the progeny includes variant progeny that have the same function or biological activity as screened in the originally transformed cells. Where distinct designations are intended, it will be clearly understood from the context as "cell", "cell line" or "cell culture".

As used herein, the term "transform" or "transformation" refers to the process of transferring a vector/nucleic acid into a host cell. If cells without strong cell wall barriers are used as host cells, transfection can be carried out, for example, by the calcium phosphate precipitation method. However, other methods for introducing DNA into cells such as nuclear injection or protoplast fusion may also be used. If prokaryotic cells or cells containing strong cell wall structures are used, then one method of transfection is calcium treatment using calcium chloride, for example.

As used herein, "expression" refers to the process by which a nucleic acid is transcribed into mRNA and/or to the process by which the mRNA obtained by transcription (also referred to as a transcript) is subsequently translated into a peptide, polypeptide or protein. The transcript and the encoded polypeptide are collectively referred to as "gene products". If a polynucleotide includes a sequence derived from genomic DNA, expression in a eukaryotic cell may include mRNA splicing.

"Vector" is a nucleic acid molecule, particularly a self-replicating one, which transfers an inserted nucleic acid molecule into and/or between host cells. The term includes vectors that are mainly used for insertion of DNA or RNA into a cell (e.g., chromosomal integration), replication vectors that are mainly used for DNA or RNA replication, and expression vectors that are used for the transcription and/or translation of DNA or RNA. Vectors that provide more than one of the aforementioned functions are also included.

"Expression vector" is a polynucleotide which, when introduced into an appropriate host cell, can be transcribed and translated into a polypeptide. "Expression system" usually refers to a suitable host cell that comprises an expression vector that can be used to produce a desired expression product.

As used herein, the term "host cell" refers to any kind of cellular system that can be engineered to produce the antibody disclosed herein. In some embodiments, HEK293 cells and CHO cells are used as host cells.

Regulatory sequences that are suitable for prokaryotes, for example, include a promoter, and optionally an operator sequence and a ribosome-binding site. Eukaryotic cells are known to utilize promoters, enhancers and polyadenylation signals.

A nucleic acid is "operably linked" when it is in a functional relationship with another nucleic acid sequence. For example, DNA for a leader sequence or secretory leader is operably linked to DNA for a polypeptide if it is expressed as a proprotein that participates in the secretion of the polypeptide; a promoter or enhancer is operably linked to a coding sequence if it affects the transcription of the sequence; or a ribosome-binding site is operably linked to a coding sequence if it is positioned so as to facilitate translation. Generally, "operably linked" means that the DNA sequences being linked are contiguous, and, in the case of a secretory leader, contiguous and in the reading frame. However, enhancers do not have to be contiguous. Similarly, in some instances, an intron may be present between nucleic acid sequences that are operably linked. Linking is accomplished by a ligation reaction at convenient restriction sites. If such sites do not exist, then synthetic oligonucleotide adaptors or adapters are used according to conventional practice.

The nucleic acid sequence encoding the antibody can be readily obtained from the literature, or by reverse translation with reference to the preferred codons of the intended host cell. The coding nucleic acid may be assembled from chemically synthesized polynucleotides and/or previously cloned antibody-encoding DNA, possibly with the help of site-directed mutagenesis.

For recombinant production of the antibody, the nucleic acid encoding it can be isolated and inserted into a replicable vector for further cloning (DNA amplification) or expression. DNA encoding the antibody can be readily isolated and sequenced using conventional procedures (e.g., by using oligonucleotide probes that are capable of binding specifically to genes encoding the heavy and light chains of the antibody). Many vectors are available. The vector components generally include, but are not limited to, one or more of the following: a signal sequence, an origin of replication, one or more marker genes, an enhancer element, a promoter, and a transcription termination sequence, for example, as described in US5,534,615, all disclosure regarding protein expression of which are specifically incorporated herein by reference.

Suitable host cells for cloning or expressing DNA in vectors herein are the prokaryotic cells, yeasts or higher eukaryotic cells described above. Suitable prokaryotes for this purpose include eubacteria, such as Gram-negative and Gram-positive organisms, for example, *Enterobacteriaceae* such as *Escherichia*, e.g., *E. coli*, *Enterobacter*, *Erwinia*, *Klebsiella*, *Proteus*, *Salmonella* such as *S*. *typhimurium*, *Serratia* such as *S*. *marcescans* and *Shigella*, and *Bacilli* such as *B. subtilis* and *B. licheniformis*, *Pseudomonas* such as P. *aeruginosa* and *Streptomyces.* One exemplary *E. coli* cloning host is *E. coli* 294 (ATCC 31,446), although other strains such as *E. coli* B, *E. coli* X1776 (ATCC 31,537) and *E. coli* W3110 (ATCC 27,325) are also suitable. These examples are illustrative rather than limiting.

In addition to prokaryotes, eukaryotic microorganisms such as filamentous fungi or yeasts are also suitable cloning or expression hosts for antibody-encoding vectors. *Saccharomyces cerevisiae*, a common baker's yeast, is the most commonly used yeast among lower eukaryotic host microorganisms. However, many other genera, species, and strains are commonly available and useful herein, such as *Schizosaccharomyces pombe*; *Kluyveromyces* hosts such as, e.g., *K. lactis*, *K. fragilis* (ATCC 12,424), *K. bulgaricus* (ATCC 16,045), *K. wickeramii* (ATCC 24,178), *K. Waltii* (ATCC 56,500), *K. drosophilarum* (ATCC 36,906), *K. thermotolerans* and *K. marxianus*; *Yarrowia* (EP 402,226); *Pichia pastoris* (EP 183,070); *Candida*: *Trichoderma reesia* (EP 244,234); *Neurospora crassa*; *Schwanniomyces* such as *S*. *occidentalis*, and filamentous fungi such as, e.g., *Neurospora*, *Penicillium*, *Tolypocladium* and *Aspergillus* hosts such as *A. nidulans* and *A. niger.*

Suitable host cells for the expression of glycosylated antibodies are derived from multicellular organisms, including invertebrate cells such as plant and insect cells. Many baculoviral strains and variants and corresponding permissive insect host cells such as *Spodoptera frugiperda* (caterpillar), *Aedes aegypti* (mosquito), *Aedes albopictus* (mosquito), *Drosophila melanogaster* (fruitfly) and *Bombyx mori* have been identified. Various viral strains for transfection are publicly available, e.g., the L-1 variant of *Autographa californica* NPV and the Bm-5 strain of *B. mori* NPV, and such viruses can be used as the virus herein according to the present invention, particularly for the transfection of *Spodoptera frugiperda* cells. Plant cell cultures of cotton, corn, potato, soybean, petunia, tomato, and tobacco can also be used as hosts.

However, interest has been greatest in vertebrate cells, and propagation of vertebrate cells in culture (tissue culture) has become a routine procedure. Examples of useful mammalian host cell lines include monkey kidney CV1 line transformed by SV40 (COS-7, ATCC CRL 1651); human embryonic kidney line (293 or 293 cells subcloned for growth in suspension culture); baby hamster kidney cells (BHK, ATCC CCL 10); Chinese hamster ovary cells/-DHFR (CHO); mouse sertoli cells (TM4); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor cells (MMT 060562, ATCC CCL51); TRI cells; MRC 5 cells; and FS4 cells.

Host cells are transformed with the expression vectors described above for antibody production and cultured in modified conventional nutrient media suitable for inducing promoters, selecting transformants or amplifying the genes encoding the desired sequences.

The host cells used to produce the antibody can be cultured in a variety of media. Commercially available media such as Ham's F10, Minimal Essential Medium (MEM), RPMI-1640 and Dulbecco's Modified Eagle Medium (DMEM) are suitable for culturing the host cells. In addition, culture media as disclosed in US4,767,704; US4,657,866; US4,927,762; US4,560,655; or US5,122,469; WO 90/03430; WO 87/00195; or US Re. 30,985 can be used as culture media for the host cells. Any of these media can be supplemented as necessary with hormones and/or other growth factors (such as insulin, transferrin or epidermal growth factors), salts (such as sodium chloride, calcium chloride, magnesium chloride, and phosphates), buffers (such as HEPES), nucleotides (such as adenosine and thymidine), antibiotics (such as Gentamycin^{™}), trace elements (defined as inorganic compounds usually present at final concentrations in micromolar order), and glucose or an equivalent energy source. Any other necessary supplements can also be included at proper concentrations, which are known to one skilled in the art. The culture conditions, such as temperature, pH, and the like, are those previously selected for the host cell for expression, and are apparent to those of ordinary skill.

When using recombinant techniques, the antibody can be produced intracellularly or in the periplasmic space, or directly secreted into the medium. If the antibody is produced intracellularly, as a first step, the particulate debris, either from host cells or from lysed fragments, can be removed, for example, by centrifugation or ultrafiltration.

The antibody composition prepared from cells can be purified by, for example, hydroxylapatite chromatography, gel electrophoresis, dialysis and affinity chromatography, and affinity chromatography is the preferred purification technique. The suitability of protein A as an affinity ligand depends on the type and isotype of any immunoglobulin Fc domain present in the antibody. Protein A can be used to purify antibodies based on human γ1, γ2 or γ4 heavy chain. Protein G is recommended for all mouse isotypes and for human γ3. The matrix to which the affinity ligand is attached is most often agarose, but other matrices are also available. Mechanically stable matrices such as controlled porous glass or poly(styrenedivinyl)benzene allow for faster flow rates and shorter operation times than can be achieved with agarose. When the antibody comprises a C_{H3} domain, the Bakerbond ABX^{™} resin can be used for purification. Other techniques for protein purification, such as fractionation on an ion-exchange column, ethanol precipitation, reversed-phase HPLC, silica chromatography, heparin SEPHAROSE^{™} chromatography, chromatography on an anion or cation exchange resin (such as a polyaspartic acid column), chromatofocusing, SDS-PAGE, and ammonium sulfate precipitation are also useful depending on the antibody to be recovered.

Following any preliminary purification step(s), the mixture comprising the antibody of interest and contaminants can be subjected to low pH hydrophobic interaction chromatography using an elution buffer having a pH of about 2.5-4.5, preferably performed at a low salt concentration (e.g., from about 0-0.25 M salt).

Once purified, the antibody can be dissolved in an aqueous solvent including any pharmaceutically acceptable buffer, salt or other excipient. The dissolved antibody can be refrigerated or frozen before use. Alternatively, it can be lyophilized and reconstituted shortly before use.

### Toxin

The toxin used for conjugation to the antibody generally should meet the following three conditions: (1) the action mechanism is clear, such as mitotic arrest, causing DNA damage, and the like; (2) it is highly active: in general, EC₉₀ is less than 1 nmol·L⁻¹; (3) it can be conjugated by chemical methods and the highly active toxin itself or a highly active derivative thereof is released in tumor cells.

The toxin used in the present invention may be any toxin commonly used in the art, and may be, for example, a microtubule inhibitor, a DNA damaging agent, and other toxin molecules.

In some embodiments, the toxin of the present invention is selected, for example, from maytansines, hemiasterlins, amanitins, auristatins, calicheamicins, duocarmycins, and the like, and deuterated forms thereof.

Maytansines are cytotoxins widely used in ADCs. They arrest the cell cycle in the G2/M phase by blocking the polymerization of tubulin, thereby inhibiting the progression of mitosis of cells and causing apoptosis.

Derivatives of maytansines can be, for example, DM1, DM4, and deuterated forms thereof.

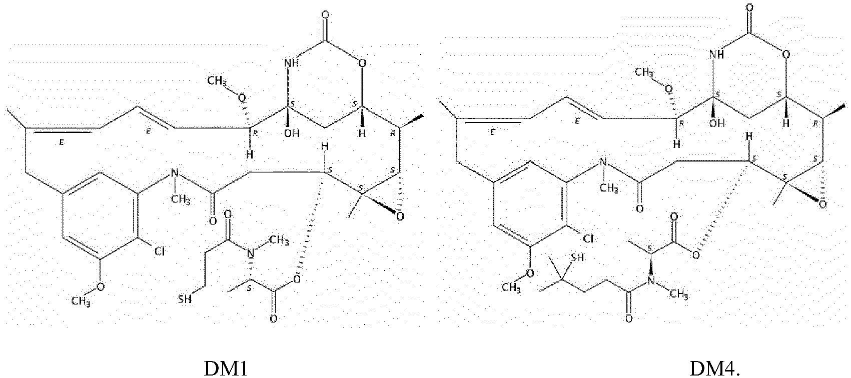

Hemiasterlins are a class of tripeptides obtained by modification of the original natural product hemiasterlin. They are inhibitors of tubulin polymerization and can bind non-competitively to the vinblastine site of tubulin.

Derivatives of hemiasterlins can be, for example, E7974 (Eisai), HTI-286 (Wyeth), and deuterated forms thereof.

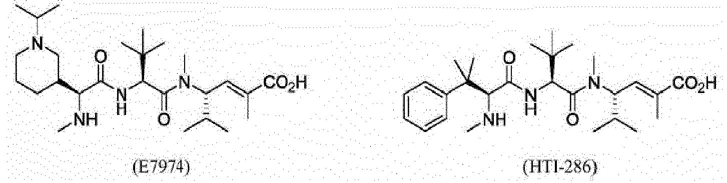

Amanitins are bicyclic octapeptides which are extracted from poisonous mushrooms and bind efficiently to mammalian RNA polymerase II. They kill cells by terminating DNA transcription. Such molecules are highly hydrophilic and have highly selective toxicity.

Amanitins may be, for example, di-deoxy variants of α-amanitin, β-amanitin, γ-amanitin, ε-amanitin, amanullin or amanullinic acid, or mono-deoxy variants of amanin, amaninamide, γ-amanin, γ-amaninamide, or the like, and deuterated forms thereof.

Auristatins are synthetic derivatives of dolastoxin 10. They achieve effective mitotic inhibition by inhibiting tubulin polymerization. Auristatins may be, for example, monomethyl auristatin E (MMAE), monomethyl auristatin F (MMAF), auristatin EB (AEB), auristatin EVB (AEVB), auristatin F phenylenediamine (AFP), and deuterated forms thereof.

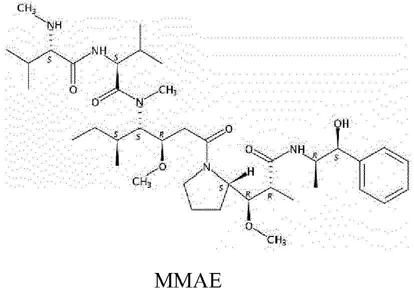

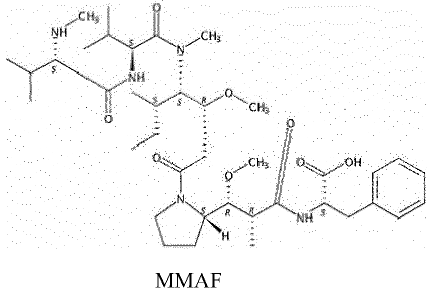

Calicheamicins have 7 major derivatives, of which calicheamicin γ is the most active and used in clinic. Calicheamicins contain three moieties of: an oligosaccharide, calicheamicinone and methyltrithio. The methyltrithio moiety acts as an initiator, which upon reduction initiates the Bergman rearrangement reaction and results in DNA cleavage.

Duocarmycins can specifically recognize the DNA minor groove and effectively alkylate the DNA base adenine at position N3. They have very high anti-cancer activity. Duocarmycin toxins can be, for example, duocarmycin A, adozelesin, CC-1065, and deuterated forms thereof.

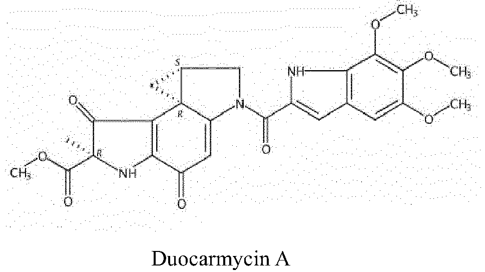

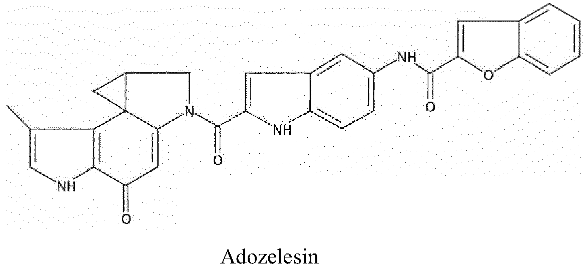

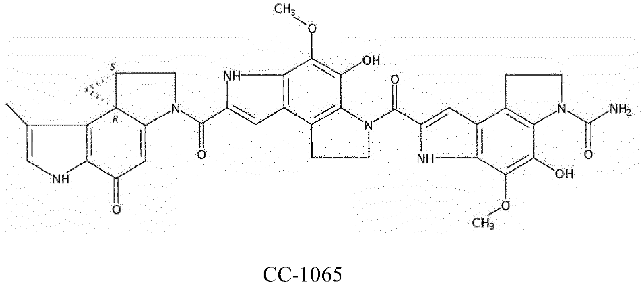

Toxins for use in the present invention may also be, for example, duocarmycins, doxorubicins (such as morpholino-doxorubicin and cyanomorpholino-doxorubicin), dolastatin, dolestatin-10, combretastatin, calicheamicins, tubulysins, disorazole, epothilones, paclitaxel, docetaxel, SN-38, topotecan, rhizoxin, echinomycin, colchicine, vinblastine, vindesine, estramustine, cemadotin, eleutherobin, methotrexate, methopterin, dichloro methotrexate, 5-fluorouracil, 6-mercaptopurine, mercaptopurine, melphalan, vinleurosine, leurosideine, actinomycin, daunorubicin and daunorubicin conjugates, mitomycin C, mitomycin A, carminomycin, aminopterin, tallysomycin, podophyllotoxins (such as etoposide or etoposide phosphate), vincristine, camptothecin, and the like, and deuterated forms thereof.

In some embodiments, the toxin of the present invention is selected from maytansines and deuterated forms thereof, such as DM1, DM4, and deuterated forms thereof.

In some embodiments, the toxin of the present invention is selected from hemiasterlins and deuterated forms thereof, such as E7974, HTI-286, and deuterated forms thereof.

In some embodiments, the toxin of the present invention is selected from amanitins and deuterated forms thereof, such as α-amanitin, β-amanitin, γ-amanitin, ε-amanitin, and the like, and deuterated forms thereof.

In some embodiments, the toxin of the present invention is selected from auristatins and deuterated forms thereof, including but not limited to, monomethyl auristatin E (MMAE), monomethyl auristatin F (MMAF), auristatin EB (AEB), auristatin EVB (AEVB), auristatin F phenylenediamine (AFP), and deuterated forms thereof.

In some embodiments, the toxin of the present invention is monomethyl auristatin F (MMAF) or a deuterated form thereof. In some embodiments, the toxin of the present invention is monomethyl auristatin E (MMAE) or a deuterated form thereof.

In some embodiments, the toxin of the present invention is selected from calicheamicins and deuterated forms thereof, such as calicheamicin γ and deuterated forms thereof.

In some embodiments, the toxin of the present invention is selected from duocarmycins and deuterated forms thereof, such as duocarmycin A, adozelesin, CC-1065, and deuterated forms thereof.

In some embodiments, the toxin of the present invention is selected from, for example, maytansines (such as DM1 and DM4), hemiasterlins (such as E7974 and HTI-286), amanitins (such as α-amanitin, β-amanitin, γ-amanitin and ε-amanitin), auristatins (such as MMAE, MMAF, AEB, AEVB and AFP), calicheamicins (such as calicheamicin γ), duocarmycins (such as duocarmycin A, adozelesin and CC-1065), and the like, and deuterated forms thereof.

### Linker

In the bispecific antibody-drug conjugate of the present invention, the linking of the bispecific antibody to the toxin is achieved by a linker. The linker of the present invention may be monofunctional, linking a single toxin molecule to a single site on the antibody; it may also be multifunctional, linking two/more toxin molecules to a single site on the antibody, or linking one toxin molecule to two/more sites on the antibody.

The linker of the antibody-drug conjugate should meet at least the following two conditions: (1) it is stable enough *in vivo* so as not to fall off the toxin in blood circulation, and thus toxicity arising from toxin falling off is avoided; and (2) the toxin is effectively released at the target.

A linker can be divided into a cleavable linker and a non-cleavable linker. One or more hydrogen atoms in the linker are optionally deuterated. The cleavable linker further includes a chemically cleavable linker and an enzymatically cleavable linker.

In some embodiments, the linker of the present invention is a cleavable linker or a deuterated form thereof. The cleavable linker is generally susceptible to cleavage, such as cleavage by lysosome, under intracellular conditions.

In some embodiments, the linker of the present invention is a chemically cleavable linker or a deuterated form thereof.

In some embodiments, the linker of the present invention is an enzymatically cleavable linker or a deuterated form thereof.

The enzymatically cleavable linker may be, for example, a linker comprising a peptide component that comprises two or more amino acids and is cleavable by an intracellular protease, such as a lysosomal protease or an endosomal protease. The peptide component may comprise naturally occurring amino acid residues, and/or minor amino acids, and/or non-naturally occurring amino acid analogs, such as citrulline. The peptide component can be designed and optimized for enzymatic cleavage by a particular enzyme, for example, a tumor-associated protease, cathepsin B, C or D, or a plasmin protease.

The enzymatically cleavable linker may be a dipeptide-containing linker, e.g., a linker containing valine-citrulline (Val-Cit) or phenylalanine-lysine (Phe-Lys). Other examples of suitable dipeptides for inclusion in the linker include Val-Lys, Ala-Lys, Me-Val-Cit, Phe-homoLys, Phe-Cit, Leu-Cit, Ile-Cit, Trp-Cit, Phe-Arg, Ala-Phe, Val-Ala, Met-Lys, Asn-Lys, Ile-Pro, Ile-Val, Asp-Val, His-Val, Met-(D)Lys, Asn-(D)Lys, Val-(D)Asp, NorVal-(D)Asp, Ala-(D)Asp, Me3Lys-Pro, phenyl Gly-(D)Lys, Met-(D)Lys, Asn-(D)Lys, Pro-(D)Lys and Met-(D)Lys. The cleavable linker may also include a longer peptide component such as a tripeptide, a tetrapeptide, or a pentapeptide. Examples include, but are not limited to, tripeptides Met-Cit-Val, Gly-Cit-Val, (D)Phe-Phe-Lys and (D)Ala-Phe-Lys, and tetrapeptides Gly-Phe-Leu-Gly and Ala-Leu-Ala-Leu.

In some embodiments, the linker of the present invention is selected from valine-citrulline (Val-Cit) or phenylalanine-lysine (Phe-Lys), and deuterated forms thereof. In some embodiments, the linker of the present invention is valine-citrulline (Val-Cit) or a deuterated form thereof.

In some embodiments, the linker of the present invention is valine-citrulline (Val-Cit, Vc) and the toxin is MMAE, i.e., the linker-toxin of the present invention is Vc-MMAE. The structure of Vc-MMAE (mc-vc-PAB-MMAE, Maleimidocaproyl-valine-citrulline-p-aminobenzyloxycarbonyl monomethylauristatin E, Vedotin) is shown below.

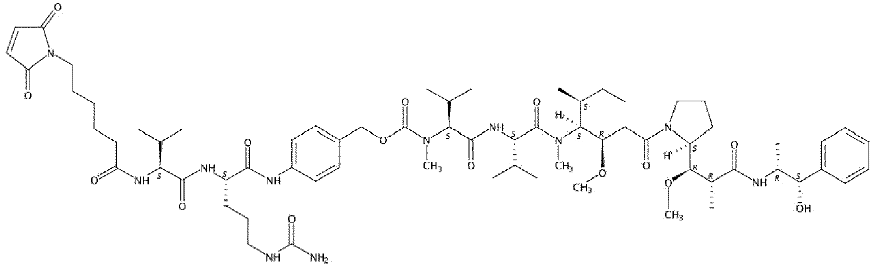

The linker blocks and the toxin MMAE of Vc-MMAE (mc-vc-PAB-MMAE) of the present invention are commercially available.

The "me" described in the present invention refers to 6-maleimidohexanoic acid, or a group formed by linking it to the bispecific antibody and/or the linker by a chemical bond.

The structural formula of 6-maleimidohexanoic acid (mc) is:

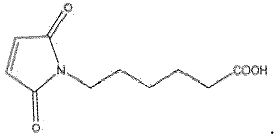

The "PAB" described in the present invention refers to p-aminobenzyl alcohol, or a group formed by linking it to the linker and/or the toxin by a chemical bond.

The structural formula of p-aminobenzyl alcohol (PAB) is:

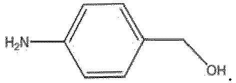

### Bispecific Antibody-Drug Conjugate

In the bispecific antibody-drug conjugate of the present invention, the antibody may be any bispecific antibody as described above; the linker may be any linker as described above, for example, a dipeptide-containing linker, such as a linker containing valine-citrulline (Val-Cit) or phenylalanine-lysine (Phe-Lys); the toxin may be any toxin as described above, such as DM1, DM4, MMAE, MMAF, and the like.

In some embodiments, the average drug-to-antibody ratio (DAR) of the bispecific antibody-drug conjugate is about 1-6. In some embodiments, the average drug-to-antibody ratio (DAR) of the bispecific antibody-drug conjugate is, for example, about 1, 2, 3, 4, 5 or 6. In some embodiments, the DAR of the bispecific antibody-drug conjugate is about 2, 3, 4 or 5. In some embodiments, the DAR of the bispecific antibody-drug conjugate is 3-4. In some embodiments, the DAR of the bispecific antibody-drug conjugate is about 3 or 4. In some embodiments, the DAR of the bispecific antibody-drug conjugate is about 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, or the like.

The structure of the bispecific antibody-drug conjugate can be represented by Formula (I): 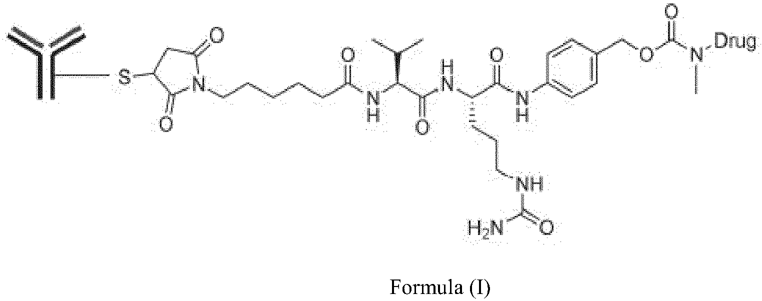 wherein 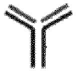 represents the bispecific antibody, the middle part is the linker moiety, valine-citrulline (Val-Cit, Vc), and the rightmost Drug represents the toxin moiety.

The free thiol on a reduced antibody can interact with a maleimide group (such as 6-maleimidohexanoic acid (mc)), thereby achieving the linking of the antibody to the linker (Val-Cit, Vc).

The linker is linked to the rightmost Drug (the toxin moiety) by p-aminobenzyl alcohol.

The bispecific antibody of Formula (I) 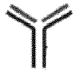 is a bispecific antibody as described above.

In some embodiments, the DAR value (n) is further indicated in the structure of the bispecific antibody-drug conjugate as shown in Formula (I'): 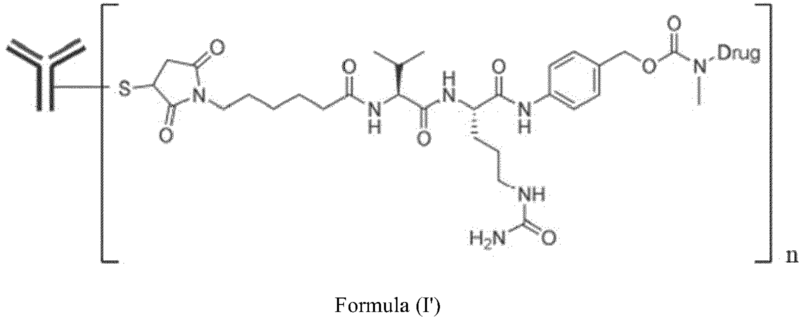 wherein 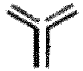 represents the bispecific antibody described above, and the middle part is the linker moiety, valine-citrulline (Val-Cit, abbreviated as Vc); the linker is linked to the bispecific antibody by 6-maleimidohexanoic acid and to the rightmost Drug (the toxin moiety) by p-aminobenzyl alcohol; n represents the average drug-to-antibody ratio (DAR), and n is selected from 1-6, e.g., about 1, 2, 3, 4, 5 or 6, e.g., about 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9 or 5.

The structure of the bispecific antibody-drug conjugate can be represented by Formula (II): 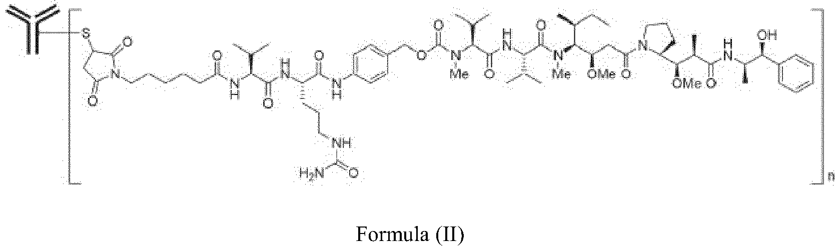 wherein 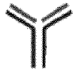 represents the bispecific antibody, and the bracketed structure is the linker-toxin moiety, Vc-MMAE; n (i.e., the average drug-to-antibody ratio, DAR) may be, for example, about 1-6, e.g., 1, 2, 3, 4, 5 or 6, e.g., 3-4, e.g., 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, or the like.

In some embodiments, the average drug-to-antibody ratio (DAR) of the bispecific antibody-drug conjugate is 3-4, e.g., 3, 3.1, 3.2, 3.3, 3.4, 3.5, 3.6, 3.65, 3.7, 3.75, 3.8, 3.9 or 4.

The free thiol on a reduced antibody can interact with a maleimide group (such as 6-maleimidohexanoic acid (mc)), thereby achieving the linking of the antibody to the linker (Val-Cit, Vc).

The linker is linked to the rightmost toxin, monomethyl auristatin E (MMAE), by p-aminobenzyl alcohol.

The bispecific antibody 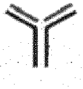 in Formula (II) is a bispecific antibody as described above.

In some embodiments, the bispecific antibody-drug conjugate of the present invention may be T51-MMAE, T52-MMAE, T53-MMAE, T54-MMAE, T55-MMAE, T56-MMAE, T57-MMAE or T58-MMAE. The bispecific antibodies T51, T52, T53, T54, T55, T56, T57 and T58 of these bispecific antibody-drug conjugates may be defucosylated (i.e., T51-AF, T52-AF, T53-AF, T54-AF, T55-AF, T56-AF, T57-AF and T58-AF), and the corresponding bispecific antibody-drug conjugates are T51-AF-MMAE, T52-AF-MMAE, T53-AF-MMAE, T54-AF-MMAE, T55-AF-MMAE, T56-AF-MMAE, T57-AF-MMAE and T58-AF-MMAE.

The "T54-MMAE" described in the present invention refers to a bispecific antibody-drug conjugate, the specific structure of which is T54-(mc-vc-PAB-MMAE)n, where n is the average drug-to-antibody ratio (DAR); T54, mc, vc, PAB and MMAE are as defined above.

The "T54-AF-MMAE" described in the present invention refers to a bispecific antibody-drug conjugate, the specific structure of which is T54-AF-(mc-vc-PAB-MMAE)n, where n is the average drug-to-antibody ratio (DAR); T54-AF, mc, vc, PAB and MMAE are as defined above.

T51-MMAE, T52-MMAE, T53-MMAE, T55-MMAE, T56-MMAE, T57-MMAE and T58-MMAE are understood similarly to "T54-MMAE".

T51-AF-MMAE, T52-AF-MMAE, T53-AF-MMAE, T55-AF-MMAE, T56-AF-MMAE, T57-AF-MMAE and T58-AF-MMAE are understood similarly to "T54-AF-MMAE".

### Preparation of Bispecific Antibody-Drug Conjugates

The ADCs of the present disclosure can be prepared by one of several routes known in the art using organic chemical reactions, conditions and reagents known to one skilled in the art (see, e.g., Bioconjugate Techniques (G. T. Hermanson, 2013, Academic Press, and the examples provided herein). For example, conjugation can be achieved in the following ways: (1) reacting a nucleophilic or electrophilic group of an antibody with a bifunctional linker to form an antibody-linker intermediate via a covalent bond, and then reacting the intermediate with an activated toxin (such as DM1, DM4, MMAE, MMAF, or the like); or (2) reacting a nucleophilic or electrophilic group of a toxin with a linker to form linker-toxin via a covalent bond, and then reacting the intermediate with a nucleophilic or electrophilic group of an antibody.

As described above, the toxin can be conjugated to various groups on the antibody via an appropriate linker to afford an ADC. For example, conjugation may be performed via antibody surface lysines, or via oxidized carbohydrates or via cysteine residues that have been released by reduction of one or more interchain disulfide bonds. Alternatively, the antibody can be modified to include other cysteine residues or include non-naturally occurring amino acids that provide reactive handles, such as selenomethionine, p-acetylphenylalanine, formylglycine or p-azidomethyl-L-phenylalanine. Such modifications are well known in the art (see, e.g., U.S. Pat. Nos. 7,521,541; 8,455,622 and 9,000,130; Hofer et al., Biochemistry, 48:12047-12057 (2009); Axup et al., PNAS, 109:16101-16106 (2012); Wu et al., PNAS, 106:3000-3005 (2009); Zimmerman et al., Bioconj. Chem., 25:351-361(2014)).

In some embodiments, the ADCs of the present disclosure comprise an auristatin toxin (such as MMAE or MMAF) conjugated via an appropriate linker to a cysteine residue on the bispecific antibody that has been released by reduction of one or more interchain disulfide bonds.

For conjugation to the cysteine residue, partial reduction of the interchain disulfide bonds of the antibody may be performed, followed by conjugation to linker-toxin. Suitable reductants are known in the art and include, for example, dithiothreitol (DTT), tris(2-carboxyethyl)phosphine (TCEP), 2-mercaptoethanol, cysteamine and many water-soluble phosphines.

The average DAR of the ADCs may be determined by standard techniques such as UV/VIS spectroscopic analysis, ELISA-based techniques, chromatography techniques such as hydrophobic interaction chromatography (HIC), UV-MALDI mass spectrometry (MS) and MALDI-TOF MS. In addition, the distribution of drug-linked forms (e.g., the percentage of DAR0, DAR1, DAR2, etc. species) may also be analyzed by various techniques known in the art, including MS (with or without an accompanying chromatographic separation step), hydrophobic interaction chromatography, reversed-phase HPLC or isoelectric focusing gel electrophoresis (IEF) (see, e.g., Sun et al., Bioconj Chem., 28:1371-81 (2017); Wakankar et al., mAbs, 3:161-172 (2011)).

In some embodiments, the average DAR of the ADCs is determined by hydrophobic interaction chromatography (HIC) techniques.

Following conjugation, the ADCs can be purified and separated from unconjugated reactants and/or any conjugate aggregates by purification methods known in the art. Such methods include, but are not limited to, size exclusion chromatography (SEC), hydrophobic interaction chromatography (HIC), ion exchange chromatography, chromatofocusing, ultrafiltration, centrifugal ultrafiltration, and combinations thereof.

The present invention also relates to a pharmaceutical composition comprising the bispecific antibody-drug conjugate described above.

The terms "treat" and "treatment" include: (1) preventing or delaying the development of at least one clinical or sub-clinical symptom of a state, disorder or condition in a subject that may be afflicted with or predisposed to the state, disorder or condition but has not experienced or displayed clinical or sub-clinical symptoms of the state, disorder or condition; or (2) inhibiting the state, disorder or condition, i.e., arresting, reducing or delaying the development of the disease or a relapse thereof (in the case of maintenance treatment) or at least one clinical or sub-clinical symptom thereof; or (3) relieving the disease, i.e., causing regression of the state, disorder or condition or at least one of its clinical or sub-clinical symptoms. The benefit to a subject to be treated is statistically significant or at least perceptible to the patient or the physician.

As used herein, the term "therapeutically effective" or "effective" with reference to dose or amount refers to an amount of a compound or pharmaceutical composition that, when administered to a subject for treating (e.g., preventing or ameliorating) a state, disorder or condition, is sufficient to cause such treatment or prevention to produce an effective result. "Therapeutically effective amount" will vary depending on the compound or bacteria or analogs administered, as well as the disease and its severity, and the age, weight, physical condition and responsiveness of the mammal to be treated.

The phrase "pharmaceutically acceptable", as used in conjunction with the composition of the present disclosure, means that molecular entities and other ingredients of such composition are physiologically tolerable and, when administered to a mammal (e.g., a human), usually do not produce adverse effects.

The term "pharmaceutical formulation" or "pharmaceutical composition" refers to a formulation or composition that is in a form that allows the biological activity of active ingredient(s) contained therein to be effective, and that contains no additional components which have unacceptable toxicity to a subject to which the formulation would be administered.

The term "carrier" refers to a diluent, adjuvant, excipient or vehicle with which the compound is administered. Such pharmaceutical carriers may be sterile liquids, such as water and oils, including those of petroleum, animal, vegetable or synthetic origin, such as peanut oil, soybean oil, mineral oil, sesame oil, and the like. Water or aqueous solution or saline solutions and aqueous glucose and glycerol solutions are used as carriers particularly for injectable solutions. Alternatively, the carrier may be a solid dosage form carrier, including but not limited to, one or more binders (for compressed pills), glidants, encapsulating agents, flavoring agents and colorants. Suitable pharmaceutical carriers are described in "*Remington's Pharmaceutical Sciences*" by E. W. Martin.

Other embodiments relate to a use of a bispecific antibody-drug conjugate or a pharmaceutical composition comprising the bispecific antibody-drug conjugate as disclosed herein for preventing and/or treating cancer. In another embodiment, provided is a use of a bispecific antibody-drug conjugate in the preparation of a medicament for preventing and/or treating cancer. In some embodiments, the use is for treating cancer. Other similar embodiments are methods for preventing and/or treating cancer, comprising administering to a patient in need thereof a therapeutically effective amount of a bispecific antibody-drug conjugate. In some embodiments, the subject is a mammal. In some embodiments, the subject is a human.

The cancer described herein is, for example, a HER2-expresing cancer, e.g., a cancer with a moderate-to-low level of HER2 expression, such as but not limited to, HER2⁺ breast cancer, ovarian cancer, endometrial cancer, cervical cancer, gastric cancer, esophageal cancer, lung cancer, head and neck cancer, bladder cancer, bile duct cancer, and/or colorectal cancer.

The bispecific antibody-drug conjugate of the present invention can be administered alone or in combination with other agents suitable for cancer treatment. For example, the bispecific antibody-drug conjugate can be used in combination with other therapies, such as chemotherapy or targeted therapy, or immunotherapy. Similarly, they can be used in combination with radiotherapy or surgery.

The bispecific antibody-drug conjugate can be delivered by any suitable route. In some embodiments, the conjugate is delivered by injection or infusion, by a dermal patch, by a reservoir/pump device, or by inhalation. In various aspects of these embodiments, the conjugate is administered intravenously, intraperitoneally, subcutaneously, or intramuscularly.

The conjugates in some embodiments can be administered once a day or less frequently. For example, in some embodiments, the bispecific antibody-drug conjugate is administered once every two days, once every three days, once every four days, once every five days, once every six days, once a week, once every two weeks, once every three weeks, once every four weeks, once a month, once every two months, once every three months, or once every six months.

The optimal dosage level of the active molecule will depend on a variety of factors, including the age, weight, physical condition of the patient, possible combinations with other drugs, and the severity of the condition. The specific dosage can be determined by one skilled in the art based on the specific circumstances.

### Kits, Unit Dosage Forms, and Articles of Manufacture

The present disclosure also provides kits, unit dosage forms, and articles of manufacture comprising the conjugate or the composition thereof described herein. The kits, unit dosage forms and articles of manufacture may include, for example, vials (such as sealed vials), pre-filled syringes, and auto-injectors (pens) of the conjugate described herein.

Amino acids are referred to herein by their commonly known 3-letter symbols or 1-letter (single-letter) symbols according to the IUPAC-IUB Biochemical Nomenclature Commission.

### Examples

The technical solutions of the present invention are described below using specific examples, and the above description of the present invention is further explained in detail, although the above subject matter of the present invention should not be understood as being limited to the following examples. All techniques implemented based on the above description of the present invention fall within the scope of the present invention.

### Example 1. Preparation of Bispecific Antibodies

CHO-K1 cells were co-transfected with bispecific antibody heavy chain plasmids and bispecific antibody light chain plasmids by electrotransfection. The cells were resuspended in a fresh opti-medium (glutamine-free, commercially available) and seeded into a 96-well plate, and were incubated in a 37 °C, 5% CO₂ incubator for 24 h for recovery. After the 24 hours' recovery, the cells were resuspended in a fresh opti-medium containing 25 µM MSX. The medium was changed for a fresh one every 3 days. The culturing was performed continuously for 2-4 weeks under the 25 µM MSX stress, and viable mixed cell clones were picked out. The mixed cells were seeded into a 96-well plate with a fresh opti-medium at 0.5 cells/well by limiting dilution and cultured in a 37 °C, 5% CO₂ incubator for about 2 weeks. Clones capable of expressing the antibody were detected by ELISA. After two rounds of screening by limiting dilution, single clones capable of expressing the antibody T54 were finally obtained.

According to the method described in the Chinese patent application CN201610194325.7, CHO-K1 cells were subjected to a knockout of the FUT8 gene with TALEN and to LCA stress to construct a fucose knockout cell strain D57. The D57 cell strain and a method similar to that for preparing T54 in Example 1 were used to prepare a defucosylated bispecific antibody T54-AF.

After the stable lines for the bispecific antibodies were cultured for days, the supernatants were subjected to protein A affinity purification and cation exchange purification to give the bispecific antibodies T54 and T54-AF.

By using a method similar to that described above in Example 1, CHO-K1 cells were co-transfected with bispecific antibody heavy chain plasmids and bispecific antibody light chain plasmids, and the antibodies T51, T51-AF, T52, T52-AF, T53, T53-AF, T55, T55-AF, T56, T56-AF, T57, T57-AF, T58 and T58-AF were prepared.

### Example 2. Preparation of Bispecific Antibody-Drug Conjugates

The bispecific antibody T54 obtained in Example 1 was dialyzed against a conjugation buffer (25 mM Na₂B₄O₇/25 mM NaCl, 1 mM DTPA, pH 7.4), and concentrated by ultrafiltration to a concentration greater than 5 mg/mL. Then the concentration was adjusted to 5 mg/mL. The reductant TCEP was added, and a reaction was conducted in a 25 °C water bath for 2 h.

The linker blocks and the toxin MMAE of Vc-MMAE (mc-vc-PAB-MMAE) are commercially available.
"mc block" refers to 6-maleimidohexanoic acid, the structural formula of which is: 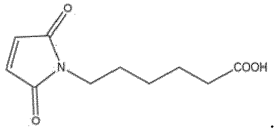 "PAB" refers to p-aminobenzyl alcohol (PAB), the structural formula of which is:

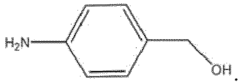

The chemical structural formula of Vc-MMAE (mc-vc-pab-MMAE) is shown below:

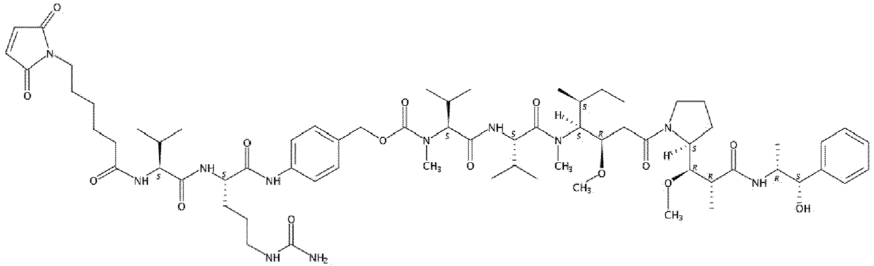

The chemical conjugation of the blocks can be accomplished using a conventional esterification or dehydration reaction in the art.
mc-vc-pab-MMAE was dissolved in DMSO to 10 mM. mc-vc-pab-MMAE and the antibody were mixed at a molar ratio of 10:1, and the mixture underwent a conjugation reaction in a 25 °C water bath for 2 h.

The conjugate was dialyzed against a dialysis buffer (20 mM His-acetate, pH 5.5), and filtered through a 0.22 µm membrane to give the bispecific antibody-drug conjugate T54-MMAE.

By using a method similar to that described above in Example 2, the bispecific antibody-drug conjugates T54-AF-MMAE, T51-MMAE, T51-AF-MMAE, T52-MMAE, T52-AF-MMAE, T53-MMAE, T53-AF-MMAE, T55-MMAE, T55-AF-MMAE, T56-MMAE, T56-AF-MMAE, T57-MMAE, T57-AF-MMAE, T58-MMAE and T58-AF-MMAE were prepared.

### Example 3. Characterization of Bispecific Antibodies T54 and T54-AF and Bispecific Antibody-Drug Conjugates T54-MMAE and T54-AF-MMAE

SEC purity analyses and HIC-HPLC analyses of the bispecific antibodies T54 and T54-AF and the bispecific antibody-drug conjugates T54-MMAE and T54-AF-MMAE were performed.

The instrument and conditions used for the SEC purity analyses were as follows:
instrument: Thermo Ultimate 3000;
column: Waters, XBridge BEH200Å SEC 3.5 µm (7.8 × 300 mm);
mobile phase: 15% isopropanol in PBS, pH 7.4;
flow rate: 0.5 mL/min, 30 min;
loading amount: 40 µg.

The instrument and conditions used for the HIC-HPLC analyses were as follows:
column: Thermo MAbPac HIC-Butyl;
mobile phases:
   mobile phase A: 50 mM Na₃PO₄, 1.5 M (NH₄)₂SO₄, 5% isopropanol, pH 6.95;
   mobile phase B: 50 mM Na₃PO₄, 5% isopropanol, pH 6.95;
   flow rate: 0.5 mL/min;
   loading amount: 100 µg.

### 1. Results for bispecific antibodies and their conjugates

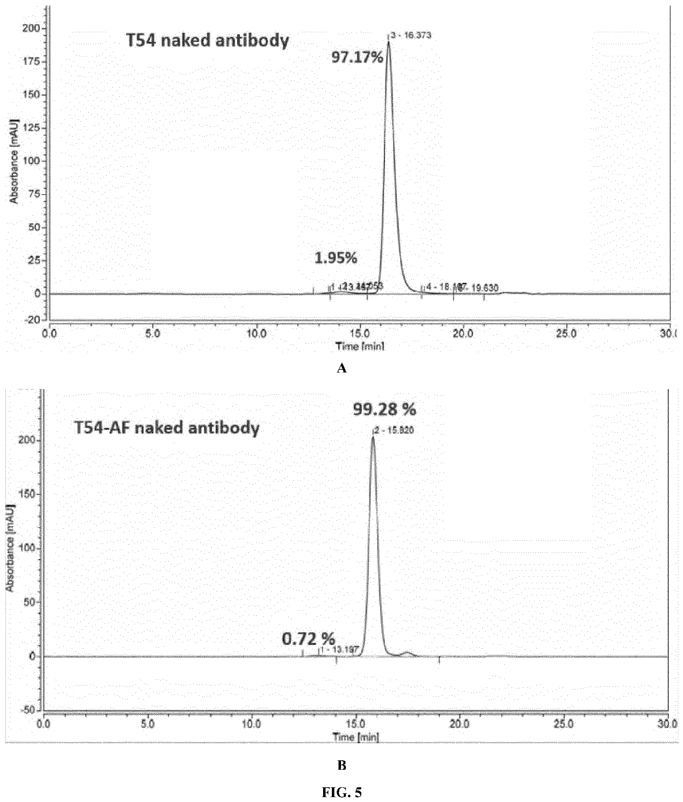

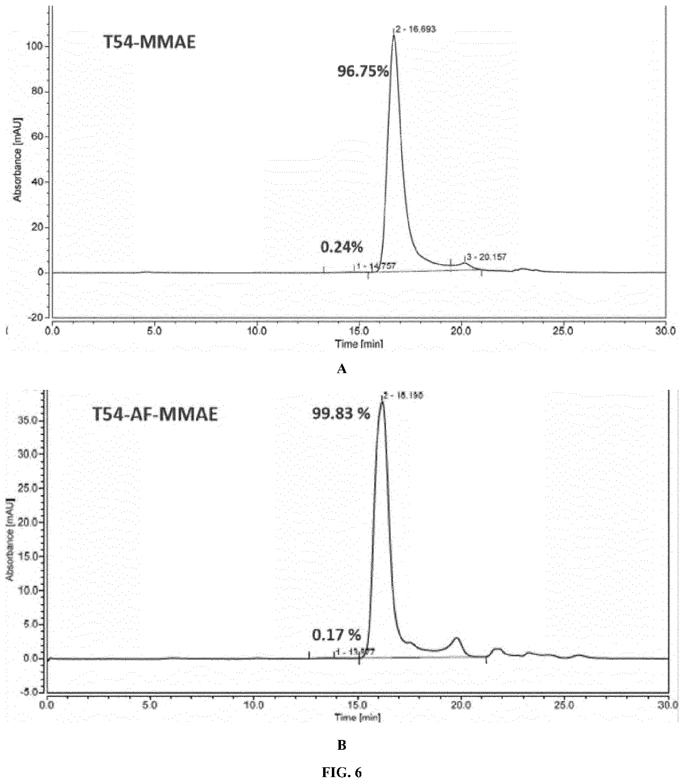

SEC purity analyses of the antibody T54 and its conjugate T54-MMAE were performed, and the results are shown in FIG. 5A and FIG. 6A, respectively. As can be seen from the figures, the polymer contents of T54 and T54-MMAE are 1.95% and 0.24%, respectively, and their monomer contents are 97.17% and 96.75%, respectively, which are close.

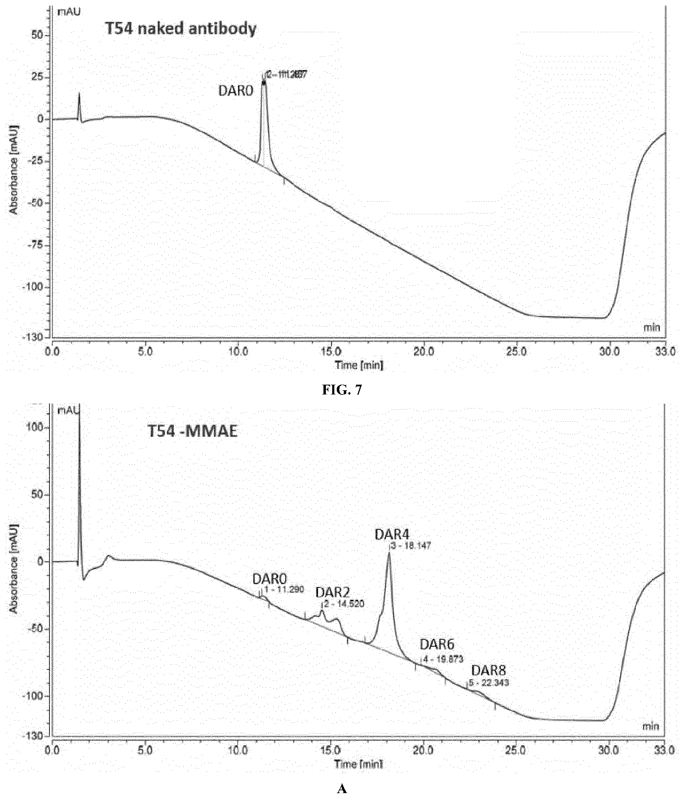

HIC-HPLC analyses of the antibody T54 and its conjugate T54-MMAE were performed, and the results are shown in FIG. 7 and FIG. 8A, respectively. As can be seen from the figures, the antibody-drug conjugates T54-MMAE contain different numbers of conjugated toxins: the proportion of those with 4 conjugated toxins is 73.31%, and the average DAR value is 3.70. The specific data are shown in Table 1-1.

**Table 1-1. The proportions of the conjugates T54-MMAE with different DAR values**

| | DAR₀ (%) | DAR₂ (%) | DAR₄ (%) | DAR₆ (%) | DAR₈ (%) | Average DAR |
|---|---|---|---|---|---|---|
| T54-MMAE | 1.29 | 20.29 | 73.31 | 2.58 | 2.53 | 3.70 |

### 2. Results for defucosylated bispecific antibodies and their conjugates

SEC purity analyses of the antibody T54-AF and its conjugate T54-AF-MMAE were performed, and the results are shown in FIG. 5B and FIG. 6B, respectively. As can be seen from the figures, the polymer contents of T54-AF and T54-AF-MMAE are 0.72% and 0.17%, respectively, and their monomer contents are 99.28% and 99.83%, respectively, which are close.

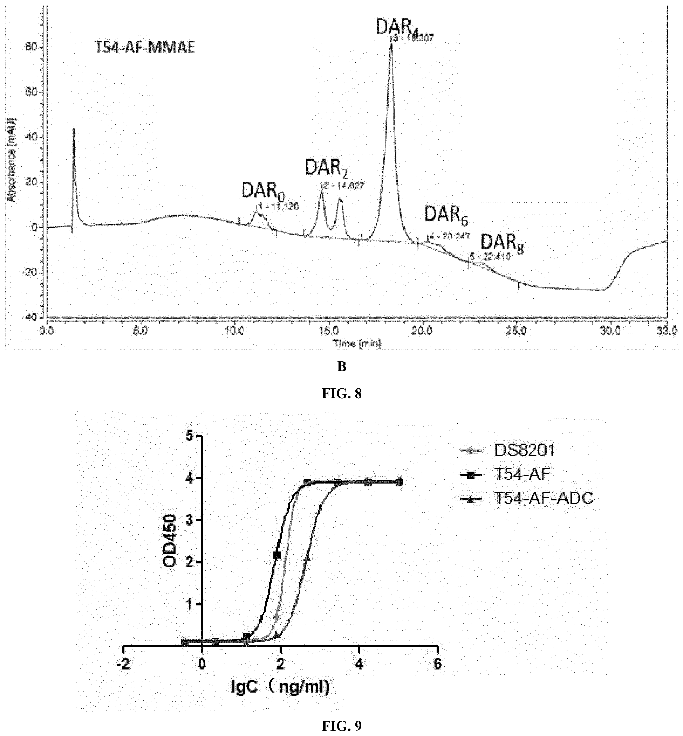

The antibody-drug conjugate T54-AF-MMAE was subjected to a HIC-HPLC analysis for the number of conjugated toxins, and the results are shown in FIG. 8B. As can be seen from the figure, the antibody-drug conjugates T54-AF-MMAE contain different numbers of conjugated toxins: the proportion of those with 4 conjugated toxins is 66.61%, and the average DAR value is 3.46. The specific data are shown in Table 1-2.

**Table 1-2. The proportions of the conjugates T54-AF-MMAE with different DAR values**

| | DAR₀ (%) | DAR₂ (%) | DAR₄ (%) | DAR₆ (%) | DAR₈ (%) | Average DAR |
|---|---|---|---|---|---|---|
| T54-AF-MMAE | 5.81 | 22.34 | 66.61 | 3.39 | 1.85 | 3.46 |

### Example 4. Assay for Binding Activity to HER2

Objects to be analyzed: the antibody T54-AF;
the antibody-drug conjugate T54-AF-MMAE;
a positive control ADC DS8201 (Trastuzumab Deruxtecan, a Her2-targeting antibody-drug conjugate, obtained by conjugating the humanized anti-Her2 antibody trastuzumab to a topoisomerase-I inhibitor camptothecin derivative (DX-8951 derivative DXd) by a tetrapeptide (GGFG) linker).

The binding activity of the antibody and the antibody-drug conjugates to the antigen HER2 was tested by ELISA. A plate was coated with the antigen HER2 (2 µg/mL) at 100 µL/well and left overnight at 4 °C. The plate was washed with PBS (300 µL × 4), blocked with 300 µL of 3% BSA in PBS at 37 °C for 2 h, and washed with PBS four times. 100 µL of the antibody to be tested was added, and the plate was incubated at 37 °C for 1 h. The plate was washed with PBS four times. 100 µL of a diluted secondary antibody was added, and the plate was incubated at 37 °C for 1 h. The secondary antibody was discarded, and the plate was washed with PBS four times. 100 µL of TMB chromogenic solution was added, and the plate was incubated at 37 °C for 5-10 min. 50 µL of dilute sulfuric acid stop solution was added to stop the reaction. OD₄₅₀ was measured with a microplate reader.

Data were analyzed. The binding activity curves of the antibody T54-AF and the antibody-drug conjugates T54-AF-MMAE and DS8201 to the HER2 antigen are shown in FIG. 9, and EC₅₀ values are shown in Table 2.

**Table 2. EC₅₀ values for the binding of the antibody/antibody-drug conjugate to the HER2 antigen**

| | T54-AF | T54-AF-MMAE | DS8201 |
|---|---|---|---|
| EC₅₀ (ng/ml) | 71.04 | 430.7 | 130.4 |

### Example 5. Inhibition of Proliferation of Tumor Cells Positive for HER2 Expression (SKBR3, N87)

The tumor cells positive for HER2 expression in the logarithmic growth phase (SKBR3, N87) were washed with PBS and trypsinized. After trypsin was neutralized with a 10% FBS medium (DMEM, RPMI1640), the cells were centrifuged, and target cells were resuspended in a corresponding 10% FBS medium (DMEM, RPMI1640). The cell density was adjusted to 2E4, and the cells were added to a 96-well plate at 100 µL/well. After 24 h of culture, drugs diluted with the medium to different concentrations were added at 100 µL/well (initial concentration 300 µg/mL, 8-fold dilution, 8 concentrations). After 72 h, the cell supernatant was removed, and 100 µL of a medium supplemented with cck8 was added. The plate was incubated for 4 h, and OD₄₅₀ was measured.

### 1. Results for bispecific antibodies and their conjugates

### (1) Inhibition of growth of breast cancer cell SKBR3

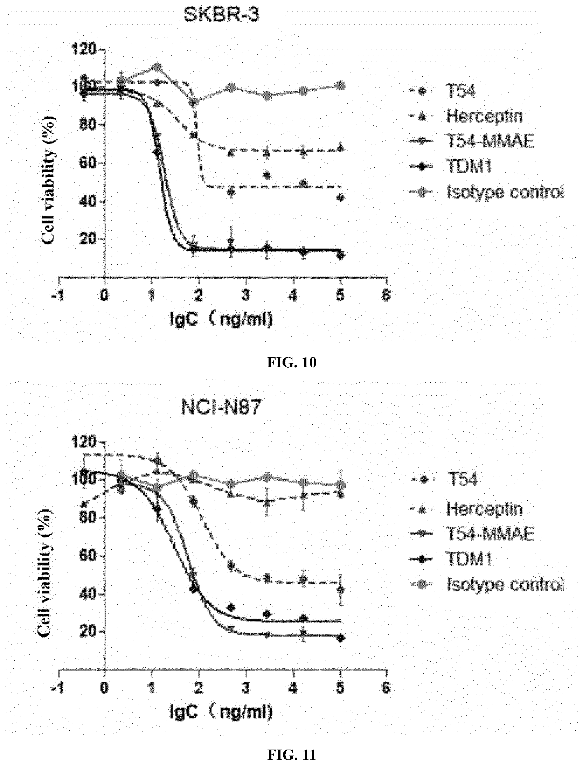

Data were analyzed using GraphPad 5.0 software. The inhibition curves of the antibodies and antibody-drug conjugates on the breast cancer cell SKBR3 are shown in FIG. 10, and IC₅₀ values are shown in Table 3. The results show that T54, T54-MMAE, TDM1 and Herceptin all have the effect of inhibiting the growth of SKBR3 tumor cells. The inhibition of tumor cells by the antibody-drug conjugates TDM1 and T54-MMAE was more significant and the maximum inhibition rate was greater than 80%. T54 was less effective than TDM1 and T54-MMAE and the maximum inhibition rate was 52.51%. Herceptin was the least effective of them all and the maximum inhibition rate was only 33.54%.

**Table 3. IC₅₀ values and maximum inhibition rates of the antibodies/antibody-drug conjugates inhibiting the growth of the breast cancer cell SKBR3**

| | T54 | T54-MMAE | Herceptin | TDM1 |
|---|---|---|---|---|
| IC₅₀(ng/ml) | 90.57 | 18.63 | 36.7 | 14.73 |
| Maximum inhibition rate (%) | 52.51% | 85.32% | 33.54% | 86.06% |

### (2) Inhibition of growth of gastric cancer cell N87

Data were analyzed using GraphPad 5.0 software. The inhibition curves of the antibodies and antibody-drug conjugates on the gastric cancer cell N87 are shown in FIG. 11, and IC₅₀ values are shown in Table 4. The results show that T54, T54-MMAE and TDM1 have the effect of inhibiting the growth of N87 tumor cells. The inhibition of tumor cells by the antibody-drug conjugates TDM1 and T54-MMAE was more significant and the maximum inhibition rate was greater than 70%. T54 was less effective than TDM1 and T54-MMAE and the maximum inhibition rate was 55.6%. Herceptin has no significant inhibitory effect on N87.

**Table 4. IC₅₀ values and maximum inhibition rates of the antibodies/antibody-drug conjugates inhibiting the growth of the gastric cancer cell N87**

| | T54 | T54-MMAE | Herceptin | TDM1 |
|---|---|---|---|---|
| IC₅₀(ng/ml) | 118.1 | 59.74 | / | 24.49 |
| Maximum inhibition rate (%) | 55.6% | 82.73% | / | 74..86% |

### 2. Results for defucosylated bispecific antibodies and their conjugates

The tumor cells in the logarithmic growth phase were washed with PBS and trypsinized. After trypsin was neutralized with a corresponding medium (DMEM, RPMI1640, MEM, etc.) containing 10% FBS, the cells were centrifuged, and target cells were resuspended in a corresponding 10% FBS medium (DMEM, RPMI1640, MEM, etc.). The cell density was adjusted to 4 × 10⁴/mL, and the cells were added to a 96-well plate at 100 µL/well. After 24 h of culture, drugs diluted with the medium to different concentrations were added at 100 µL/well (initial concentration 100 µg/mL, 6-fold dilution, 8 concentrations). After 72 h, the cell supernatant was removed, and 100 µL of a medium supplemented with cck8 was added. The plate was incubated for 4 h, and OD₄₅₀ was measured.

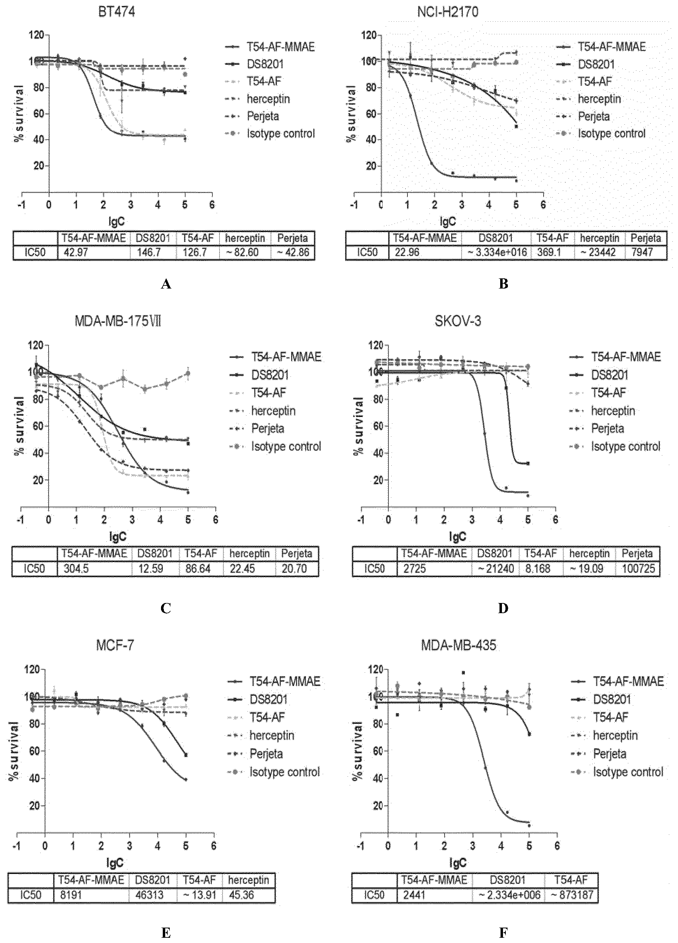

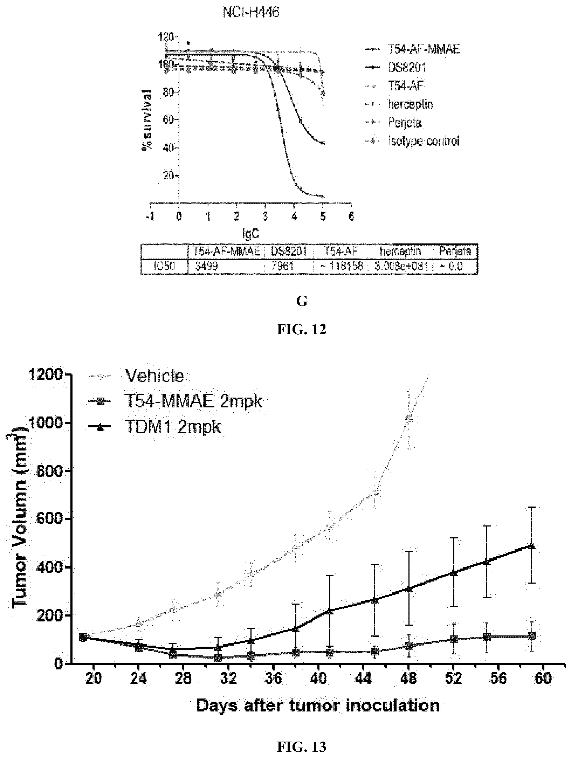

The inhibitory activity of T54-AF-MMAE, DS8201, T54-AF, Herceptin, Perjeta and Isotype (isotype control, as a negative antibody) on the proliferation of tumor cells with high, moderate and low levels of HER2 expression was tested, and data were analyzed. The results are shown in FIG. 12: T54-AF-MMAE has an essentially better inhibitory effect on the growth of different tumor cells positive for HER2 expression than DS8201 and the T54-AF, Herceptin and Perjeta mAbs.

### Example 6. Inhibition of Proliferation of BT474 Breast Cancer Tumor Cells in Mice

### 1. Results for bispecific antibody-drug conjugates

### (1) Cell culture

BT474 tumor cells were purchased from ATCC, under the number HTB-20^{™}. The tumor cells were cultured in an RPMI 1640 medium containing inactivated 10% fetal bovine serum, 1 mM Napyr, 1× MEM-NEAA, 1× MEM VITAMIN and 10 µg/mL human insulin in a 37 °C, 5% CO₂ incubator. Every 3 to 4 days when cells were confluent, they were passaged by dividing the cell culture into flasks. The tumor cells in the logarithmic growth phase were used for *in vivo* tumor inoculation.

### (2) Tumor cell inoculation and grouping

BT474 tumor cells were resuspended in PBS + Matrigel (1:1) to a concentration of 1 × 10⁸/mL, and the suspension was inoculated subcutaneously into the right anterior rib part of experimental animals at 100 µL/animal. When tumors grew to an average volume of about 111 mm³, animals were divided into 3 groups of 5 and administered. The specific administration regimen is shown in Table 5-1.

**Table 5-1. Administration regimen**

| Group | Number of animals | Treatment | Dose (mg/kg) | Route of administration | Frequency of administration |
|---|---|---|---|---|---|
| 1 | 5 | Solvent control | - | i.p. | qw x 2 |
| 2 | 5 | TDM1 | 2 | i.p. | qw x 2 |
| 3 | 5 | T54-MMAE | 2 | i.p. | qw x 2 |

| | | | | | |
|---|---|---|---|---|---|
| qw x 2 denotes: administered once a week for 2 weeks | | | | | |

### (3) Measurement of mouse weight and experimental indicators

The long and short diameters of tumors were measured using a vernier caliper, and tumor volume was calculated according to the formula: volume = 0.5 × long diameter × short diameter². The measurement and calculation were performed twice a week. The body weight of mice was measured while measuring tumor volume. The relationship between the change in the body weight of mice and the time of administration was recorded. At the same time, the survival and healthy condition of the mice, such as animal activity, eating and other general conditions during the administration, were observed.

T/C values were calculated from tumor volume, where T is the mean of relative tumor volume (RTV) of a treatment group, and C is the mean of relative tumor volume (RTV) of the solvent control group. RTV is the ratio of tumor volume after administration to that before administration. Tumor growth inhibition rate (%) = (1 - T/C) × 100%. A tumor growth inhibition rate of ≥ 60% and p < 0.05 from statistical analysis indicate the drug is effective.

### (4) Sampling and processing

After the experiment ended, the mice in each group were euthanized, and tumor tissues were removed, weighed, placed in order, and photographed.

The tumor growth curve and the tumor growth inhibition rate for each group of mice are shown in FIG. 13 and Table 6-1. TDM1 (2 mg/kg) and T54-MMAE (2 mg/kg) showed strong anti-tumor effects in the human xenograft breast cancer model BT474 and they effectively inhibited tumor growth. The tumor inhibition effect of T54-MMAE is better than that of TDM1. Tumor-bearing mice did not lose weight after administration as compared with before administration, showing good tolerance for the test substances.

**Table 6-1. The tumor inhibition effects (tumor volume) of bispecific antibody-drug conjugates on the human xenograft breast cancer model BT474**

| Group | Number of animals (after/before administration) | Body weight (g)^{a} | | Tumor volume (mm³)^{a} | | T/C (%) | Tumor growth inhibition rate (%) |
|---|---|---|---|---|---|---|---|
| | | Before administration (D19) | After administration (D59) | Before administration (D19) | After administration (D59) | | |
| Group 1: solvent control | 5/5 | 22.5 ± 0.3 | 25.4 ± 0.8 | 111 ± 12 | 1981 ± 431 | - | - |
| Group 2: TDM1 2 mg/kg | 5/5 | 22.2 ± 0.5 | 23.8 ± 0.2 | 111 ± 13 | 493 ± 157 | 25% | 75% |
| Group 3: T54-MMAE 2 mg/kg | 5/5 | 21.9 ± 0.3 | 23.4 ± 0.3 | 110 ± 12 | 67 ± 15 | 4% | 96% |

### 2. Results for defucosylated bispecific antibody-drug conjugates

### (1) Cell culture

BT474 cells were cultured in an RPMI-1640 medium containing inactivated 10% fetal bovine serum, 100 U/mL penicillin and 100 µg/mL streptomycin as well as 2 mM glutamine in a 37 °C, 5% CO₂ incubator. Every 3 to 4 days when cells were confluent, they were passaged by dividing the cell culture into flasks. The tumor cells in the logarithmic growth phase were used for *in vivo* tumor inoculation.

### (2) Tumor cell inoculation and grouping

The tumor cells were washed with PBS twice and then resuspended in PBS : Matrigel (mixed at 1:1, v/v), and the cell concentration was adjusted to 1 × 10⁸/mL. The suspension was inoculated subcutaneously into the right anterior rib part of experimental animals at 100 µL/mouse, i.e., 1 × 10⁷/mouse. From the day before inoculation, experimental animals were injected subcutaneously with estradiol benzoate injectable (2 mL: 4 mg, Ningbo Second Hormone Factory) at 40 µg/animal, once a week. When tumors grew to a mean tumor volume of 113 mm³, animals were grouped, according to tumor volume, into 7 groups of 5 and administered. The specific administration regimen is shown in Table 5-2.

**Table 5-2. Administration regimen**

| Group | Number of animals | Test compound | Dose (mg/kg, mpk) | Route of administration | Frequency of administration |
|---|---|---|---|---|---|
| G1 | 5 | Vehicle | - | i.p. | qw × 4 |
| G2 | 5 | T54-AF-MMAE | 4 | i.p. | qw × 4 |
| G3 | 5 | T54-AF-MMAE | 1 | i.p. | qw × 4 |
| G4 | 5 | T54-AF-MMAE | 0.25 | i.p. | qw × 4 |
| G5 | 5 | DS8201 | 4 | i.p. | qw × 4 |
| G6 | 5 | DS8201 | 1 | i.p. | qw × 4 |
| G7 | 5 | DS8201 | 0.25 | i.p. | qw × 4 |

| | | | | | |
|---|---|---|---|---|---|
| qw x 4 denotes: administered once a week for 4 weeks | | | | | |

### (3) Measurement of mouse weight and experimental indicators

The long and short diameters of tumors were measured using a vernier caliper, and tumor volume was calculated according to the formula: volume = 0.5 × long diameter × short diameter². The measurement and calculation were performed twice a week. The body weight of mice was measured while measuring tumor volume. The relationship between the change in the body weight of mice and the time of administration was recorded. At the same time, the survival and healthy condition of the mice, such as animal activity, eating and other general conditions during the administration, were observed.

T/C values were calculated from tumor volume, where T is the mean of relative tumor volume (RTV) of a treatment group, and C is the mean of relative tumor volume (RTV) of the solvent control group. RTV is the ratio of tumor volume after administration to that before administration. Tumor growth inhibition rate (%) = (1 - T/C) × 100%. A tumor growth inhibition rate of ≥ 60% and *p* < 0.05 from statistical analysis indicate the drug is effective.

### (4) Sampling and processing

After the experiment ended, the mice in each group were euthanized, and tumor tissues were removed, weighed, placed in order, and photographed.

### (5) Results

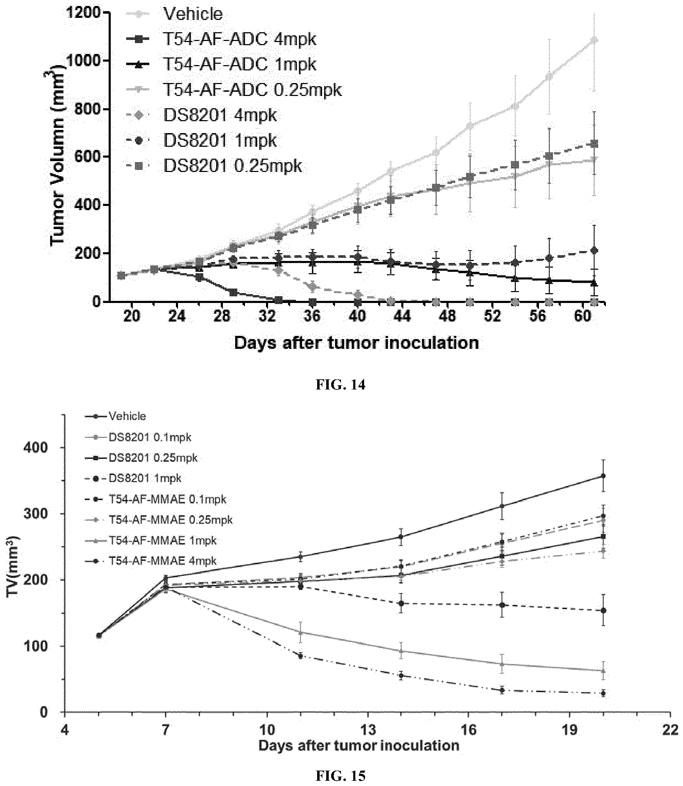

The tumor growth curve and post-administration tumor growth inhibition for each group of mice are shown in FIG. 14, Table 6-2, continued Table 6-2-1 and continued Table 6-2-2. The results show that, in the human xenograft breast cancer model BT474,
1) compared to the blank control (Vehicle), T54-AF-MMAE 4mpk began to show a significant tumor inhibition effect at D26; T54-AF-MMAE 1mpk began to show a significant tumor inhibition effect at D33 (7 days after the second administration);
2) T54-AF-MMAE high (4mpk) showed significant superiority over the same dose of the control drug (DS8201) at D26 (7 days after the first administration) and D33 (7 days after the second administration).

During the treatment, tumor-bearing mice showed good tolerance for high-, medium- and low-dose bispecific antibody-ADC test drugs, and each group of mice had normal body weight, behaved normally, and were in a good general condition.

**Table 6-2. The tumor inhibition effects (tumor volume) of defucosylated bispecific antibody-drug conjugates on the human xenograft breast cancer model BT474**

| Group | Number of animals (mice) | Body weight (g)^{a} | | Tumor volume (mm³)^{a} | | T/C (%) | Tumor growth inhibition rate (%) | *p^{b}* | *p^{c}* |
|---|---|---|---|---|---|---|---|---|---|
| | | Before administration (PG-D0) | After administration (PG-D7) | Before administration (PG-D0) | After administration (PG-D7) | | | | |
| Vehicle | 5 | 18.7 ± 0.4 | 19.0 ± 0.6 | 108 ± 2 | 177 ± 2 | - | - | | |
| T54-AF-MMAE, 4 mg/kg | 5 | 18.9 ± 0.5 | 18.8 ± 0.5 | 107 ± 2 | 102 ± 17 | 58 | 42 | * | ** |
| T54-AF-MMAE, 1 mg/kg | 5 | 19.0 ± 0.6 | 19.1 ± 0.5 | 107 ± 3 | 147 ± 18 | 83 | 17 | - | - |
| T54-AF-MMAE, 0.25 mg/kg | 5 | 19.4 ± 0.5 | 19.3 ± 0.4 | 106 ± 3 | 163 ± 10 | 92 | 8 | - | - |
| DS8201, 4 mg/kg | 5 | 18.9 ± 0.5 | 18.6 ± 0.6 | 107 ± 2 | 148 ± 10 | 84 | 16 | - | - |
| DS8201, 1 mg/kg | 5 | 19.0 ± 0.4 | 19.5 ± 0.3 | 107 ± 3 | 147 ± 6 | 83 | 17 | - | - |
| DS8201, 0.25 mg/kg | 5 | 18.6 ± 0.3 | 19.1 ± 0.3 | 107 ± 2 | 167 ± 11 | 94 | 6 | - | - |

| Group | Number of animals | Body weight (g)^{a} | | Tumor volume (mm³)^{a} | | T/C (%) | Tumor growth inhibition rate (%) | *p^{b}* | *p^{c}* |
|---|---|---|---|---|---|---|---|---|---|
| | | Before administration (PG-D0) | After administration (PG-D14) | Before administration (PG-D0) | After administration (PG-D14) | | | | |
| Vehicle | 5 | 18.7 ± 0.4 | 19.5 ± 0.7 | 108 ± 2 | 298 ± 23 | - | - | - | - |
| T54-AF-MMAE, 4 mg/kg | 5 | 18.9 ± 0.5 | 19.4 ± 0.5 | 107 ± 2 | 7 ± 4 | 2 | 98 | * | ** |
| T54-AF-MMAE, 1 mg/kg | 5 | 19.0 ± 0.6 | 19.4 ± 0.6 | 107 ± 3 | 165 ± 35 | 55 | 45 | * | - |
| T54-AF-MMAE, 0.25 mg/kg | 5 | 19.4 ± 0.5 | 20.4 ± 0.4 | 106 ± 3 | 275 ± 36 | 92 | 8 | - | - |
| DS8201, 4 mg/kg | 5 | 18.9 ± 0.5 | 19.5 ± 0.6 | 107 ± 2 | 131 ± 24 | 44 | 56 | - | - |
| DS8201, 1 mg/kg | 5 | 19.0 ± 0.4 | 20.2 ± 0.5 | 107 ± 3 | 184 ± 28 | 62 | 38 | - | - |
| DS8201, 0.25 mg/kg | 5 | 18.6 ± 0.3 | 19.3 ± 0.4 | 107 ± 2 | 271 ± 22 | 91 | 9 | - | - |

| Before administration (PG-D0) | After administration (PG-D21) | Before administration (PG-D0) | After administration (PG-D21) | | | | | | |
|---|---|---|---|---|---|---|---|---|---|
| Vehicle | 5 | 18.7 ± 0.4 | 19.0 ± 0.6 | 108 ± 2 | 458 ± 35 | - | - | - | - |
| T54-AF-MMAE, 4 mg/kg | 5 | 18.9 ± 0.5 | 18.8 ± 0.5 | 107 ± 2 | 0 | 0 | 100 | * | - |
| T54-AF-MMAE, 1 mg/kg | 5 | 19.0 ± 0.6 | 19.1 ± 0.5 | 107 ± 3 | 169 ± 46 | 37 | 63 | * | - |
| T54-AF-MMAE, 0.25 mg/kg | 5 | 19.4 ± 0.5 | 19.3 ± 0.4 | 106 ± 3 | 396 ± 71 | 87 | 13 | - | - |
| DS8201, 4 mg/kg | 5 | 18.9 ± 0.5 | 18.6 ± 0.6 | 107 ± 2 | 31 ± 19 | 7 | 93 | - | - |
| DS8201, 1 mg/kg | 5 | 19.0 ± 0.4 | 19.5 ± 0.3 | 107 ± 3 | 186 ± 44 | 41 | 59 | - | - |
| DS8201, 0.25 mg/kg | 5 | 18.6 ± 0.3 | 19.1 ± 0.3 | 107 ± 2 | 380 ± 47 | 83 | 17 | - | - |

| | | | | | | | | | |
|---|---|---|---|---|---|---|---|---|---|
| Notes: a. mean ± standard error; b. compared to the Vehicle group; c. compared to the same dose of the control drug; * indicates <0.001, and ** indicates <0.05. | | | | | | | | | |

### Example 7. Inhibition of Proliferation of N87 Gastric Cancer Tumor Cells in Mice

### (1) Cell culture

The tumor cells were cultured in an RPMI-1640 medium containing inactivated 10% fetal bovine serum, 100 U/mL penicillin and 100 µg/mL streptomycin as well as 2 mM glutamine in a 37 °C, 5% CO₂ incubator. Every 3 to 4 days when cells were confluent, they were passaged by dividing the cell culture into flasks. The tumor cells in the logarithmic growth phase were used for *in vivo* tumor inoculation.

### (2) Tumor cell inoculation and grouping

The tumor cells were washed with PBS twice, and the cell concentration was adjusted to 1 × 10⁸/mL. The suspension was inoculated subcutaneously into the right anterior rib part of experimental animals at 100 µL/mouse, i.e., 1 × 10⁷/mouse. When tumors grew to a mean tumor volume of 80-120 mm³, the animals were grouped, according to tumor volume, into 8 groups of 8 and administered. The specific administration regimen is shown in Table 7-1.

**Table 7-1. Administration regimen**

| Group | Number of animals | Test compound | Dose (mg/kg, mpk) | Route of administration | Frequency of administration |
|---|---|---|---|---|---|
| G1 | 8 | Vehicle | - | i.p. | qw × 4 |
| G2 | 8 | DS8201 | 0.1 | i.p. | qw × 4 |
| G3 | 8 | DS8201 | 0.25 | i.p. | qw × 4 |
| G4 | 8 | DS8201 | 1 | i.p. | qw × 4 |
| G5 | 8 | T54-AF-MMAE | 0.1 | i.p. | qw × 4 |
| G6 | 8 | T54-AF-MMAE | 0.25 | i.p. | qw × 4 |
| G7 | 8 | T54-AF-MMAE | 1 | i.p. | qw × 4 |
| G8 | 8 | T54-AF-MMAE | 4 | i.p. | qw × 4 |

| | | | | | |
|---|---|---|---|---|---|
| Notes: The administration volume is 10 µL/g animal body weight; i.p. stands for intraperitoneal injection; qw x 4 denotes: administered once a week for 4 weeks. | | | | | |

### (3) Measurement of mouse weight and experimental indicators

The long and short diameters of tumors were measured using a vernier caliper, and tumor volume was calculated according to the formula: volume = 0.5 × long diameter × short diameter². The measurement and calculation were performed twice a week. The body weight of mice was measured while measuring tumor volume. The relationship between the change in the body weight of mice and the time of administration was recorded. At the same time, the survival and healthy condition of the mice, such as animal activity, eating and other general conditions during the administration, were observed.

T/C values were calculated from tumor volume, where T is the mean of relative tumor volume (RTV) of a treatment group, and C is the mean of relative tumor volume (RTV) of the solvent control group. RTV is the ratio of tumor volume after administration to that before administration. Tumor growth inhibition rate (%) = (1 - T/C) × 100%. A tumor growth inhibition rate of ≥ 60% and p < 0.05 from statistical analysis indicate the drug is effective.

### (4) Sampling and processing

After the experiment ended, the mice in each group were euthanized, and tumor tissues were removed, weighed, placed in order, and photographed.

### (5) Results

The tumor growth curve and post-administration tumor growth inhibition for each group of mice are shown in FIG. 15 and Table 7-2. The results show that, in the human xenograft gastric cancer model N87,
1) DS8201 1mpk, T54-AF-MMAE 0.25mpk, 1mpk and 4mpk can all significantly inhibit the growth of the NCI-N87 tumor;
2) at the same dose, T54-AF-MMAE (1mpk) has a significantly better tumor inhibition effect than DS8201 (1mpk), p = 0.005 (Student's t test).

During the treatment, tumor-bearing mice showed good tolerance for high-, medium- and low-dose bispecific antibody-ADC test drugs, and each group of mice had normal body weight, behaved normally, and were in a good general condition.

**Table 7-2. The tumor inhibition effects of defucosylated bispecific antibody-drug conjugates on the human xenograft gastric cancer model N87**

| Group | Number of animals | Body weight (g)^{a} | | Tumor volume (mm³)^{a} | | T/C (%) | Tumor growth inhibition rate (%) |
|---|---|---|---|---|---|---|---|
| | | Before administration (PG-D0) | After administration (PG-D15) | Before administration (PG-D0) | After administration (PG-D15) | | |
| Vehicle | 8 | 23.0±0.5 | 23.7±0.5 | 116 ± 2 | 357±24 | - | - |
| T54-AF-MMAE, 4 mg/kg | 8 | 23.0±0.4 | 24.2±0.3 | 117 ± 2 | 29±5 | 8.0 | 92.0 |
| T54-AF-MMAE, 1 mg/kg | 8 | 23.1±0.3 | 23.9±0.5 | 116 ± 2 | 63±14 | 17.7 | 82.3 |
| T54-AF-MMAE, 0.25 mg/kg | 8 | 23.0±0.3 | 23.7±0.4 | 116 ± 2 | 243±10 | 68.1 | 31.9 |
| T54-AF-MMAE, 0.1 mg/kg | 8 | 23.4±0.6 | 24.2±0.6 | 117 ± 2 | 297±16 | 83.0 | 17.0 |
| DS8201, 1 mg/kg | 8 | 23.2±0.2 | 23.9±0.3 | 116 ± 2 | 154±23 | 43.2 | 56.8 |
| DS8201, 0.25 mg/kg | 8 | 23.0±0.6 | 23.8±0.6 | 116 ± 2 | 266±18 | 74.4 | 25.6 |
| DS8201, 0.1 mg/kg | 8 | 23.3±0.3 | 23.8±0.4 | 115 ± 2 | 290±18 | 81.9 | 18.1 |

| | | | | | | | |
|---|---|---|---|---|---|---|---|
| Note: a. mean ± standard error. | | | | | | | |

### Example 8. Structures and Sequences of Bispecific Antibodies of the Present Application

The structures and sequences of the bispecific antibodies T51-T58 referred to in the present application are shown in Tables 8 and 9, respectively.

The bispecific antibodies T51 to T58 and their corresponding defucosylated bispecific antibodies T51-AF to T58-AF comprise an M428L substitution in the Fc region to increase the serum half-life of the bispecific antibodies, although the substitution is not shown in Table 8.

**Table 8. The structures of the bispecific antibodies**

| No. | Heavy chain #1 | Heavy chain | Homologous light chain |
|---|---|---|---|
| T5 1 | Bip_mAb_HC11 | (V_{H_Pert_T30A})-C_{H1}-adapter-(V_{H_Tra_N54T/D98S})-C_{H1}-Fc | Tra_LC_{_N30S_S56Y} |
| T5 | Bip_mAb_HC12 | (V_{H_Pert_T30A/G56A})-C_{H1}-adapter-(V_{H_Tra}__{N54T/D98S})-C_{H1}- | Tra_LC_{_N30S_S56Y} |
| 2 | | Fc | |
| T5 3 | Bip_mAb_HC13 | (V_{H_Pert_T30A})-C_{H1}-Fc-adapter-(V_{H_Tra_N54T/D98S})-C_{H1} | Tra_LC__{N30S_S56Y} |
| T5 4 | 1.Bip_mAb_HC14(Knob ) | 1.(V_{H_Pert_T30A})-CH₁-(FC_{(T366W)}) | Tra_LC__{N30S_S56Y} |
| | | 2.(V_{H_Tra_N54T/D98S})-C_{H1}-(Fc_{(T366S/L368A/Y407V)}) | |
| | 2.Bip_mAb_HC15(hole) | | |
| T5 5 | Bip_mAb_HC11 | (V_{H_Pert_T30A})-C_{H1}-adapter-(V_{H_Tra_N54T/D98S})-C_{H1}-Fc | Tra_LC_{_N30S_S56Y_T94} W |
| T5 6 | Bip_mAb_HC12 | (V_{H_Pert_T30A/G56A})-C_{H1}-adapter-(V_{H_Tra_N54T/D98S})-C_{H1}-Fc | Tra_LC__{N30S}__{S56Y}__{T94} W |
| T5 7 | Bip_mAb_HC13 | (V_{H_Pert_T30A})-C_{H1}-Fc-adapter-(V_{H_Tra_N54T/D98S})-C_{H1} | Tra_LC_{_N30S_S56Y_T94} W |
| T5 8 | 1.Bip_mAb_HC14(Knob ) | 1.(V_{H_Pert_T30A})-C_{H1}-(FC_{(T366W)}) | Tra_LC_{_N30S_S56Y_T94} W |
| | | 2.(V_{H_Tra_N54T/D98S})-C_{H1}-(FC_{(T366S/L368A/Y407V)}) | |
| | 2.Bip_mAb_HC15(hole) | | |

| | | | |
|---|---|---|---|
| *Kabat numbering is used in this table except that the Eu numbering is used for substitutions in the Fc region; V_{H-Pert}: the heavy chain variable region of pertuzumab; V_{H-Tra}: the heavy chain variable region of trastuzumab; Tra-LC: the light chain of trastuzumab. | | | |

The specific amino acid sequences for the column "heavy chain #1" in Table 8 are shown in Table 9.

**Table 9. Related descriptions of the antibodies and their amino acid sequences**

| SEQ ID NO. | Description* | Amino acid sequence |
|---|---|---|
| 1 | The light chain variable domain of pertuzumab | |
| 2 | The light chain of pertuzumab | DIQMTQSPSSLSASVGDRVTITCKASQDVSIGVAWYQQKPGKAP KLLIYSASYRYTGVPSRFSGSGSGTDFTLTISSLQPEDFATYYCQ QYYIYPYTFGQGTKVEIKRTVAAPSVFIFPPSDEQLKSGTASVVC LLNNFYPREAKVQWKVDNALQSGNSQESVTEQDSKDSTYSLSS |
| | | TLTLSKADYEKHKVYACEVTHQGLSSPVTKS FNRGEC |
| 3 | The heavy chain variable domain of pertuzumab | |
| 4 | The heavy chain of pertuzumab | EVQLVESGGGLVQPGGSLRLSCAASGFTFTDYTMDWVRQAPG KGLEWVADVNPNSGGSIYNQRFKGRFTLSVDRSKNTLYLQMNS LRAEDTAVYYCARNLGPSFYFDYWGQGTLVTVSSASTKGPSVF PLAPSSKSTSGGTAALGCLVKDYFPEPVTVSWNSGALTSGVHTF PAVLQSSGLYSLSSVVTVPSSSLGTQTYICNVNHKPSNTKVDKK VEPKSCDKTHTCPPCPAPELLGGPSVFLFPPKPKDTLMISRTPEV TCVVVDVSHEDPEVKFNWYVDGVEVHNAKTKPREEQYNSTYR VVSVLTVLHQDWLNGKEYKCKVSNKALPAPIEKTISKAKGQPR EPQVYTLPPSREEMTKNQVSLTCLVKGFYPSDIAVEWESNGQPE NNYKTTPPVLDSDGSFFLYSKLTVDKSRWQQGNVFSCSVMHEA LHNHYTQKSLSLSPG |
| 5 | The light chain variable domain of trastuzumab | |
| 6 | The light chain of trastuzumab | DIQMTQSPSSLSASVGDRVTITCRASQDVNTAVAWYQQKPGKA PKLLIYSASFLYSGVPSRFSGSRSGTDFTLTISSLQPEDFATYYCQ QHYTTPPTFGQGTKVEIKRTVAAPSVFIFPPSDEQLKSGTASVVC LLNNFYPREAKVQWKVDNALQSGNSQESVTEQDSKDSTYSLSS TLTLSKADYEKHKVYACEVTHQGLSSPVTKSFNRGEC |
| 7 | The heavy chain variable domain of trastuzumab | |
| 8 | The heavy chain of trastuzumab | EVQLVESGGGLVQPGGSLRLSCAASGFNIKDTYIHWVRQAPGK GLEWVARIYPTNGYTRYADSVKGRFTISADTSKNTAYLQMNSL RAEDTAVYYCSRWGGDGFYAMDYWGQGTLVTVSSASTKGPSV FPLAPSSKSTSGGTAALGCLVKDYFPEPVTVSWNSGALTSGVHT FPAVLQSSGLYSLSSVVTVPSSSLGTQTYICNVNHKPSNTKVDK |
| | | KVEPPKSCDKTHTCPPCPAPELLGGPSVFLFPPKPKDTLMISRTP EVTCVVVDVSHEDPEVKFNWYVDGVEVHNAKTKPREEQYNST YRVVSVLTVLHQDWLNGKEYKCKVSNKALPAPIEKTISKAKGQ PREPQVYTLPPSRDELTKNQVSLTCLVKGFYPSDIAVEWESNGQ PENNYKTTPPVLDSDGSFFLYSKLTVDKSRWQQGNVFSCSVMH EALHNHYTQKSLSLSPGK |
| 9 | Homologous light chains (homologous light chains of T51-T54) | DIQMTQSPSSLSASVGDRVTITCRASQDVSTAVAWYQQKPGKAP KLLIYSASFLYYGVPSRFSGSRSGTDFTLTISSLQPEDFATYYCQQ HYTTPPTFGQGTKVEIKRTVAAPSVFIFPPSDEQLKSGTASVVCL LNNFYPREAKVEWKVDNALQSGNSQESVTEEDSKDSTYSLSST LTLSKADYEKHKVYACEVTHQGLSSPVTKSFNRGEC |
| 10 | The sequence of Bip_mAb_HC11 (including the signal sequence): the heavy chain V-region T30A of pertuzumab, then the V-region N54T and D98S of trastuzumab, having Fc M428L; Fab-Ig format | MNFGLSLIFLVLILKGVQCEVQLVESGGGLVQPGGSLRLSCAAS GFTFADYTMDWVRQAPGKGLEWVADVNPNSGGSIYNQRFKGR FTLSVDRSKNTLYLQMNSLRAEDTAVYYCARNLGPSFYFDYWG QGTLVTVSSASTKGPSVFPLAPSSKSTSGGTAALGCLVKDYFPEP VTVSWNSGALTSGVHTFPAVLQSSGLYSLSSVVTVPSSSLGTQT YICNVNHKPSNTKVDKKVEPKSCDKGGGSGGGSGGGSGGGEV QLVESGGGLVQPGGSLRLSCAASGFNIKDTYIHWVRQAPGKGL EWVARIYPTTGYTRYADSVKGRFTISADTSKNTAYLQMNSLRAE DTAVYYCSRWGGSGFYAMDYWGQGTLVTVSSASTKGPSVFPL APSSKSTSGGTAALGCLVKDYFPEPVTVSWNSGALTSGVHTFPA VLQSSGLYSLSSVVTVPSSSLGTQTYICNVNHKPSNTKVDKKVE PKSCDKTHTCPPCPAPELLGGPSVFLFPPKPKDTLMISRTPEVTC VVVDVSHEDPEVKFNWYVDGVEVHNAKTKPREEQYNSTYRV VSVLTVLHQDWLNGKEYKCKVSNKALPAPIEKTISKAKGQPRE PQVYTLPPSREEMTKNQVSLTCLVKGFYPSDIAVEWESNGQPEN NYKTTPPVLDSDGSFFLYSKLTVDKSRWQQGNVFSCSVLHEAL HNHYTQK SLSLSPGK |
| 11 | The sequence of Bip_mAb_HC12 (including the signal | MNFGLSLIFLVLILKGVQCEVQLVESGGGLVQPGGSLRLSCAAS GFTFADYTMDWVRQAPGKGLEWVADVNPNSGASIYNQRFKGR FTLSVDRSKNTLYLQMNSLRAEDTAVYYCARNLGPSFYFDYWG |
| | sequence): the heavy chain V-region T30A/G56A of pertuzumab, then the V-region N54T and D98S of trastuzumab, having Fc M428L; Fab-Ig format | QGTLVTVSSASTKGPSVFPLAPSSKSTSGGTAALGCLVKDYFPEP VTVSWNSGALTSGVHTFPAVLQSSGLYSLSSVVTVPSSSLGTQT YICNVNHKPSNTKVDKKVEPKSCDKGGGSGGGSGGGSGGGEV QLVESGGGLVQPGGSLRLSCAASGFNIKDTYIHWVRQAPGKGL EWVARIYPTTGYTRYADSVKGRFTISADTSKNTAYLQMNSLRAE DTAVYYCSRWGGSGFYAMDYWGQGTLVTVSSASTKGPSVFPL APSSKSTSGGTAALGCLVKDYFPEPVTVSWNSGALTSGVHTFPA VLQSSGLYSLSSVVTVPSSSLGTQTYICNVNHKPSNTKVDKKVE PKSCDKTHTCPPCPAPELLGGPSVFLFPPKPKDTLMISRTPEVTC VVVDVSHEDPEVKFNWYVDGVEVHNAKTKPREEQYNSTYRV VSVLTVLHQDWLNGKEYKCKVSNKALPAPIEKTISKAKGQPRE PQVYTLPPSREEMTKNQVSLTCLVKGFYPSDIAVEWESNGQPEN NYKTTPPVLDSDGSFFLYSKLTVDKSRWQQGNVFSCSVLHEAL HNHYTQKSLSLSPGK |
| 12 | The sequence of Bip_mAb_HC13 (including the signal sequence): the heavy chain T30A of pertuzumab, having Fc M428L, then the V-region N54T and D98S of trastuzumab; Ig-Fab format | MNFGLSLIFLVLILKGVQCEVQLVESGGGLVQPGGSLRLSCAAS GFTFADYTMDWVRQAPGKGLEWVADVNPNSGGSIYNQRFKGR FTLSVDRSKNTLYLQMNSLRAEDTAVYYCARNLGPSFYFDYWG QGTLVTVSSASTKGPSVFPLAPSSKSTSGGTAALGCLVKDYFPEP VTVSWNSGALTSGVHTFPAVLQSSGLYSLSSVVTVPSSSLGTQT YICNVNHKPSNTKVDKKVEPKSCDKTHTCPPCPAPELLGGPSVF LFPPKPKDTLMISRTPEVTCVVVDVSHEDPEVKFNWYVDGVEV HNAKTKPREEQYNSTYRVVSVLTVLHQDWLNGKEYKC KVSNKALPAPIEKTISKAKGQPREPQVYTLPPSREEMTKNQVSL TCLVKGFYPSDIAVEWESNGQPENNYKTTPPVLDSDGSFFLYSK LTVDKSRWQQGNVFSCSVLHEALHNHYTQKSLSLSPGGGGSGG GSGGGSGGGEVQLVESGGGLVQPGGSLRLSCAASGFNIKDTYIH WVRQAPGKGLEWVARIYPTTGYTRYADSVKGRFTISADTSKNT AYLQMNSLRAEDTAVYYCSRWGGSGFYAMDYWGQGTLVTVSS ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVEPKSC |
| 13 | The sequence of Bip_mAb_HC14 (including the signal sequence): the heavy V-region T30A of pertuzumab, having Fc "knob" for the heterodimeric format T366W and Fc M428L | MNFGLSLIFLVLILKGVQCEVQLVESGGGLVQPGGSLRLSCAAS GFTFADYTMDWVRQAPGKGLEWVADVNPNSGGSIYNQRFKGR FTLSVDRSKNTLYLQMNSLRAEDTAVYYCARNLGPSFYFDYWG QGTLVTVSSASTKGPSVFPLAPSSKSTSGGTAALGCLVKDYF PEPVTVSWNSGALTSGVHTFPAVLQSSGLYSLSSVVTVPSSSLGT QTYICNVNHKPSNTKVDKKVEPKSCDKTHTCPPCPAPELLGGPS VFLFPPKPKDTLMISRTPEVTCVVVDVSHEDPEVKFNWYVDGV EVHNAKTKPREEQYNSTYRVVSVLTVLHQDWLNGKEYKCKVS NKALPAPIEKTISKAKGQPREPQVYTLPPSREEMTKNQVSLWCL VKGFYPSDIAVEWESNGQPENNYKTTPPVLDSDGSFFLYSKLTV DKSRWQQGNVFSCSVLHEALHNHYTQKSLSLSPGK |
| 14 | The sequence of Bip_mAb_HC15 (including the signal sequence): the V-region N54T and D98S of trastuzumab, having Fc "hole" for the heterodimeric format T366S/L368A/Y407V and Fc M428L | MNFGLSLIFLVLILKGVQCEVQLVESGGGLVQPGGSLRLSCAAS GFNIKDTYIHWVRQAPGKGLEWVARIYPTTGYTRYADSVKGRF TISADTSKNTAYLQMNSLRAEDTAVYYCSRWGGSGFYAMDYW GQGTLVTVSSASTKGPSVFPLAPSSKSTSGGTAALGCLVKDYF PEPVTVSWNSGALTSGVHTFPAVLQSSGLYSLSSVVTVPSSSLGT QTYICNVNHKPSNTKVDKKVEPKSCDKTHTCPPCPAPELLGGPS VFLFPPKPKDTLMISRTPEVTCVVVDVSHEDPEVKFNWYVDGV EVHNAKTKPREEQYNSTYRVVSVLTVLHQDWLNGKEYKCKVS NKALPAPIEKTISKAKGQPREPQVYTLPPSREEMTKNQVSLSCA VKGFYPSDIAVEWESNGQPENNYKTTPPVLDSDGSFFLVSKLTV DKSRWQQGNVFSCSVLHEALHNHYTQKSLSLSPGK |
| 15 | Adapter | GGGSGGGSGGGSGGG |
| 16 | Signal sequence** | MNFGLSLIFLVLILKGVQC |

| | | |
|---|---|---|
| *V-region substitutions are indicated by Kabat numbering; Fc region substitutions are indicated by Eu numbering. **The signal sequence is used to help secretion of the antibodies from the host cell, and is subsequently removed by enzymatic cleavage. | | |

### Other Descriptions

The present application contains a sequence listing in a file named "SEQUENCE LISTING-BISPECIFIC ANTIBODY-DRUG CONJUGATE", which is incorporated herein by reference in its entirety.

Unless otherwise indicated, all numbers expressing quantities of ingredients, properties such as molecular weight, reaction conditions, and the like used in the specification and claims should be understood as being modified by the term "about" in all instances. As used herein, the terms "about" and "approximately" mean within 10 to 15%, preferably within 5 to 10%. Therefore, unless indicated to the contrary, the numerical parameters set forth in the specification and attached claims are approximations that may vary depending on the desired properties sought to be obtained by the present invention. At least and not as an attempt to limit the application of equivalents to the scope of the claims, each numerical parameter should at least be constructed according to the given significant digits and using a common rounding method. Although the numerical ranges and parameters describing the broad scope of the present invention are approximations, the numerical values given in specific examples are as precise as possible. However, any numerical value inherently contains certain errors which necessarily arise from the standard deviation present in their respective testing measurements.

Unless otherwise indicated herein or clearly contradicted by the context, the terms "a", "an", "the" and similar referents used in the context of describing the present invention (particularly in the context of the attached claims) should be construed to include both the singular and the plural. The description of ranges of numerical values herein is merely intended to serve as a shorthand method of citing each individual value falling within the range. Unless otherwise indicated herein, each individual value is incorporated into the specification as if it were individually incorporated herein. Unless otherwise indicated herein or clearly contradicted by the context, all methods described herein can be performed in any suitable order. The use of any and all examples or exemplary language (e.g., "such as") provided herein is merely intended to better illustrate the present invention and does not limit the scope of the present invention, unless otherwise indicated. No language in the specification should be construed as indicating any non-claimed element essential to the implementation of the present invention.

Groupings of alternative elements or embodiments of the present invention disclosed herein should not be construed as limitations. Each group member may be referred to and claimed individually or in any combination with other members of the group or other elements herein. It is anticipated that one or more members of a group may be included in or deleted from the group due to convenience and/or patentability. When any such inclusion or deletion occurs, the present application is deemed to contain modified groups to fulfill the written description of all Markush groups used in the attached claims.

Certain embodiments of the present invention are described herein, including the best mode known to the inventors for implementing the present invention. Of course, variations on these described embodiments will become apparent to one of ordinary skill in the art after reading the above description. The inventors anticipate that one of ordinary skill can properly use such variations, and the inventors intend for the present invention to be applied differently than specifically described herein. Therefore, the present invention includes all modifications and equivalents of the subject matter described in the claims attached herein as permitted by applicable law. Moreover, unless otherwise indicated herein or otherwise clearly indicated to the contrary by the context, any combination of the elements described above in all possible variations is encompassed by the present invention.

Particular embodiments disclosed herein may be further limited in the claims by using "consisting of" or "consisting essentially of" language. When used in the claims, whether as filed or added according to amendment, the transition term "consisting of" excludes any element, step or ingredient not specified in the claims. The transition term "consisting essentially of" limits the scope of a claim to the specified materials or steps and those that do not materially affect the basic and novel characteristic(s). The embodiments of the present invention claimed are inherently or explicitly described and achieved herein.

Moreover, numerous patents and printed publications are cited as references throughout the specification. Each of the references and printed publications described above is individually incorporated herein by reference in its entirety.

In closing, it should be appreciated that the embodiments of the present invention disclosed herein are merely used to illustrate the principles of the present invention. Other modifications that can be used fall within the scope of the present invention. Therefore, by way of example but not of limitation, alternative configurations of the present invention may be utilized according to the teachings herein. Therefore, the present invention is not limited to what has been precisely shown and described.

## Claims

1. A bispecific antibody-drug conjugate (ADC), a pharmaceutically acceptable salt thereof, a solvate thereof or a deuterated form thereof, comprising a bispecific antibody, a toxin and a linker, wherein:
the bispecific antibody comprises a first paratope capable of recognizing and/or binding to domain II of HER2, and a second paratope capable of recognizing and/or binding to domain IV of HER2; the bispecific antibody is in a Fab-Ig, Ig-Fab or heterodimeric Ig format; preferably, the bispecific antibody is in a heterodimeric Ig format;
the toxin is selected from the group consisting of maytansines, hemiasterlins, amanitins, auristatins, calicheamicins and duocarmycins;
the linker is a cleavable linker or a non-cleavable linker; the cleavable linker includes, but is not limited to a chemically cleavable linker and an enzymatically cleavable linker; the enzymatically cleavable linker includes a linker comprising a peptide component;
alternatively, one or more hydrogen atoms in the toxin and/or linker are optionally deuterated.

2. The bispecific antibody-drug conjugate, the pharmaceutically acceptable salt thereof, the solvate thereof or the deuterated form thereof according to claim 1, wherein the bispecific antibody comprises a pair of homologous light chains and a pair of heterologous heavy chains comprising two different heavy chain variable domains, wherein:
(1) the pair of homologous light chains comprise a modified trastuzumab light chain variable domain;
the modified trastuzumab light chain variable domain is based on an unmodified trastuzumab light chain variable domain and comprises the following sequence modification(s):
a) any one of S56Y/T/A;
b) any one of N30S/A;
c) any one of T94W/F/Y;
d) any one of H91Y/F/W;
e) any one of P96Y/F/W; or
f) any combination of five kinds of modifications of a) to e);
preferably, the modified trastuzumab light chain variable domain is based on an unmodified trastuzumab light chain variable domain and comprises the following sequence modification(s): any one, two or three of three kinds of modifications of N30S, S56Y and T94W;
the sequence of the unmodified trastuzumab light chain variable domain is set forth in SEQ ID NO: 5;
(2) of the two different heavy chain variable domains,
1) one is an unmodified pertuzumab heavy chain variable domain or a modified pertuzumab heavy chain variable domain, and the sequence of the unmodified pertuzumab heavy chain variable domain is set forth in SEQ ID NO: 3;
the modified pertuzumab heavy chain variable domain is based on the unmodified pertuzumab heavy chain variable domain and comprises the following sequence modification(s):
i) any one of T30A/S/N/D;
ii) any one of G56A/S/T; or
iii) any combination of two kinds of modifications of i) and ii);
preferably, the modified pertuzumab heavy chain variable domain is based on the unmodified pertuzumab heavy chain variable domain and comprises the following sequence modification(s): T30A and/or G56A;
2) the other heavy chain variable domain is an unmodified trastuzumab heavy chain variable domain or a modified trastuzumab heavy chain variable domain, and the sequence of the unmodified trastuzumab heavy chain variable domain is set forth in SEQ ID NO: 7;
the modified trastuzumab heavy chain variable domain is based on the unmodified trastuzumab heavy chain variable domain and comprises the following sequence modification(s):
i) any one of N54T/S/A;
ii) any one of D98S/W/T/R; or
iii) any combination of two kinds of modifications of i) and ii);
preferably, the modified trastuzumab heavy chain variable domain is based on the unmodified trastuzumab heavy chain variable domain and comprises the following sequence modification(s): N54T and/or D98S.

3. The bispecific antibody-drug conjugate, the pharmaceutically acceptable salt thereof, the solvate thereof or the deuterated form thereof according to claim 1 or 2, wherein the bispecific antibody is in a heterodimeric Ig format in which Fc regions of the two heterologous heavy chains are linked by a knob-into-hole structure.

4. The bispecific antibody-drug conjugate, the pharmaceutically acceptable salt thereof, the solvate thereof or the deuterated form thereof according to any of claims 1-3, wherein two Fc regions of the bispecific antibody are modified Fc regions comprising a substitution consisting of M428L.

5. The bispecific antibody-drug conjugate, the pharmaceutically acceptable salt thereof, the solvate thereof or the deuterated form thereof according to any of claims 1-4, wherein the Fc regions of the bispecific antibody include:
(1) an Fc that increases antibody-dependent cellular cytotoxicity (ADCC) effects, or
(2) an Fc that increases antibody-dependent cellular phagocytosis (ADCP), or
(3) an Fc that increases complement-dependent cytotoxicity (CDC) activity.

6. The bispecific antibody-drug conjugate, the pharmaceutically acceptable salt thereof, the solvate thereof or the deuterated form thereof according to any of claims 1-5, wherein the bispecific antibody is a defucosylated antibody; preferably, the defucosylated antibody is produced by a host cell with a fucosylation deficiency; the fucosylation deficiency is from a knockout of the FUT8 gene.

7. The bispecific antibody-drug conjugate, the pharmaceutically acceptable salt thereof, the solvate thereof or the deuterated form thereof according to any of claims 1-6, wherein:
the bispecific antibody is in a heterodimeric Ig format in which Fc regions of the two heterologous heavy chains are linked by a knob-into-hole structure;
preferably, the Fc region of the knob part is an unmodified pertuzumab heavy chain Fc region or comprises the following modification: T366W; the Fc region of the hole part is an unmodified trastuzumab heavy chain Fc region or comprises any combination of the following modifications: T366S, L368A andY407V;
most preferably, the Fc region of the knob part is based on an unmodified pertuzumab heavy chain Fc and comprises the following sequence modifications: T366W and M428L; the Fc region of the hole part is based on an unmodified trastuzumab heavy chain Fc region and comprises the following sequence modifications: T366S, L368A and Y407V; and the bispecific antibody further comprises in the Fc region of the hole part the following modification: M428L.

8. The bispecific antibody-drug conjugate, the pharmaceutically acceptable salt thereof, the solvate thereof or the deuterated form thereof according to any of claims 1-5, wherein:
the bispecific antibody is selected from the group consisting of T51, T52, T53, T54, T55, T56, T57 and T58; or
the bispecific antibody is selected from the group consisting of defucosylated T51 (T51-AF), defucosylated T52 (T52-AF), defucosylated T53 (T53-AF), defucosylated T54 (T54-AF), defucosylated T55 (T55-AF), defucosylated T56 (T56-AF), defucosylated T57 (T57-AF) and defucosylated T58 (T58-AF).

9. The bispecific antibody-drug conjugate, the pharmaceutically acceptable salt thereof, the solvate thereof or the deuterated form thereof according to any of claims 1-8, wherein:
the toxin is selected from the group consisting of DM1, DM4, E7974, HTI-286, α-amanitin, β-amanitin, γ-amanitin, ε-amanitin, amanullin, amanin, amaninamide, γ-amanin, monomethyl auristatin F, monomethyl auristatin E, auristatin EB, auristatin EVB, auristatin F phenylenediamine, calicheamicin γ, duocarmycin A, adozelesin and CC-1065, and deuterated forms thereof; preferably, the toxin is selected from the group consisting of monomethyl auristatin E, monomethyl auristatin F, and deuterated forms thereof;
the enzymatically cleavable linker is selected from the group consisting of valine-citrulline, phenylalanine-lysine, and deuterated forms thereof; preferably, the enzymatically cleavable linker is valine-citrulline or deuterated forms thereof.

10. A bispecific antibody-drug conjugate (ADC), a pharmaceutically acceptable salt thereof, a solvate thereof or a deuterated form thereof, comprising a bispecific antibody, a toxin and a linker, wherein:
(1) the bispecific antibody is selected from the group consisting of T51, T52, T53, T54, T55, T56, T57 and T58; preferably, the bispecific antibody is a defucosylated antibody: T51-AF, T52-AF, T53-AF, T54-AF, T55-AF, T56-AF, T57-AF or T58-AF;
(2) the toxin is selected from the group consisting of DM1, DM4, E7974, HTI-286, monomethyl auristatin F, monomethyl auristatin E, auristatin EB, auristatin EVB, auristatin F phenylenediamine, and deuterated forms thereof; preferably, the toxin is selected from the group consisting of monomethyl auristatin E, monomethyl auristatin F, and deuterated forms thereof;
(3) the linker is selected from the group consisting of valine-citrulline, phenylalanine-lysine, and deuterated forms thereof; preferably, the linker is valine-citrulline or deuterated forms thereof.

11. The bispecific antibody-drug conjugate, the pharmaceutically acceptable salt thereof, the solvate thereof or the deuterated form thereof according to claim 10, wherein:
the bispecific antibody-drug conjugate has a structure represented by Formula (I'): 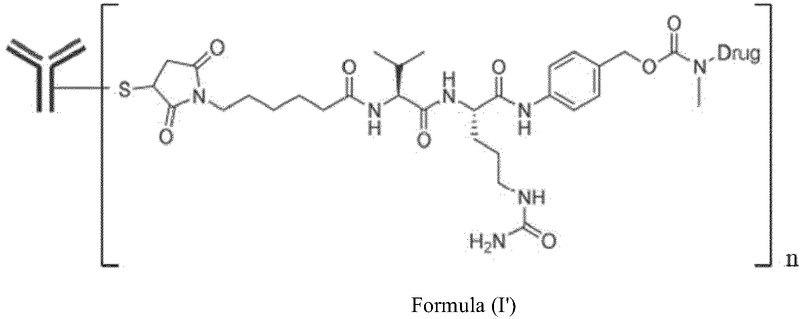 wherein:
(1) 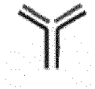 represents the bispecific antibody according to claim 10;
(2) the middle part is the linker moiety, valine-citrulline (Val-Cit, abbreviated as Vc); the linker is linked to the bispecific antibody by 6-maleimidohexanoic acid and to the rightmost Drug (the toxin moiety) by p-aminobenzyl alcohol;
the toxin is selected from the group consisting of DM1, DM4, E7974, HTI-286, monomethyl auristatin F, monomethyl auristatin E, auristatin EB, auristatin EVB, auristatin F phenylenediamine, and a deuterated form thereof;
(3) n represents an average drug-to-antibody ratio (DAR) of the bispecific antibody-drug conjugate and is selected from 1-6.

12. The bispecific antibody-drug conjugate, the pharmaceutically acceptable salt thereof, the solvate thereof or the deuterated form thereof according to claim 11, wherein:
the bispecific antibody-drug conjugate has a structure represented by Formula (II): 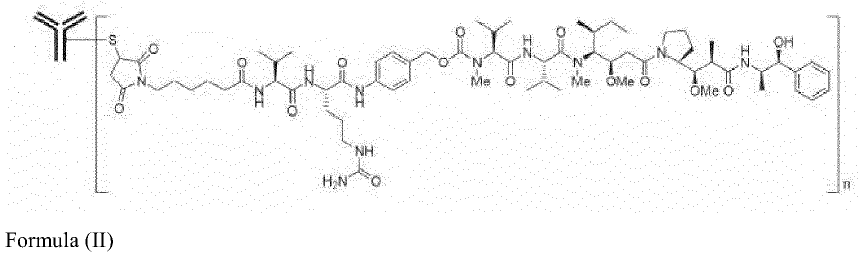 wherein:
(1) 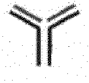 represents the bispecific antibody according to claim 10;
(2) the bracketed structure is the linker-toxin moiety, Vc-MMAE; Vc is linked to the bispecific antibody by 6-maleimidohexanoic acid and to the toxin monomethyl auristatin E (MMAE) by p-aminobenzyl alcohol;
(3) n is a DAR value selected from 1-6.

13. The bispecific antibody-drug conjugate, the pharmaceutically acceptable salt thereof, the solvate thereof or the deuterated form thereof according to any of claims 10-12, wherein the bispecific antibody is selected from the group consisting of T54, T58, defucosylated T54 (T54-AF) and defucosylated T58 (T58-AF).

14. A pharmaceutical composition comprising the bispecific antibody-drug conjugate, the pharmaceutically acceptable salt thereof, the solvate thereof or the deuterated form thereof according to any of claims 1-13, and a pharmaceutically acceptable carrier or excipient.

15. A use of the bispecific antibody-drug conjugate, the pharmaceutically acceptable salt thereof, the solvate thereof or the deuterated form thereof according to any of claims 1-13 or the pharmaceutical composition according to claim 14 in the preparation of a medicament for preventing and/or treating cancer.

16. The use according to claim 15, wherein the cancer is a HER2-expressing cancer; preferably, the cancer is a cancer with a moderate-to-low level of HER2 expression; preferably, the cancer comprises breast cancer, ovarian cancer, endometrial cancer, cervical cancer, gastric cancer, esophageal cancer, lung cancer, head and neck cancer, bladder cancer, bile duct cancer, and colorectal cancer.

17. A use of the bispecific antibody-drug conjugate, the pharmaceutically acceptable salt thereof, the solvate thereof or the deuterated form thereof according to any of claims 1-13 or the pharmaceutical composition according to claim 14 in the preparation of an anti-tumor combined formulation, wherein the combined formulation further comprises the following optional active ingredients for treating tumors:
(1) a drug that targets a target other than HER2;
(2) a cell-therapy drug; and
(3) an immunotherapy drug.

18. A method for preventing and/or treating cancer, comprising administering to a patient in need thereof a therapeutically effective amount of the bispecific antibody-drug conjugate, the pharmaceutically acceptable salt thereof, the solvate thereof or the deuterated form thereof according to any of claims 1-13 or the pharmaceutical composition according to claim 14.

19. The method according to claim 18, wherein the cancer is a HER2-expressing cancer; preferably, the cancer is a cancer with a moderate-to-low level of HER2 expression; preferably,
the cancer includes breast cancer, ovarian cancer, endometrial cancer, cervical cancer, gastric cancer, esophageal cancer, lung cancer, head and neck cancer, bladder cancer, bile duct cancer, and colorectal cancer.

20. A use of the bispecific antibody-drug conjugate, the pharmaceutically acceptable salt thereof, the solvate thereof or the deuterated form thereof according to any of claims 1-13 or the pharmaceutical composition according to claim 14 in the treatment of cancer, wherein the bispecific antibody-drug conjugate, the pharmaceutically acceptable salt thereof, the solvate thereof or the deuterated form thereof, or the pharmaceutical composition, is used in combination with the following drugs or treatment regimens for treating cancer:
(1) a drug that targets a target other than HER2;
(2) a cell-therapy drug;
(3) an immunotherapy drug;
(4) a surgery; and
(5) a physical therapy such as radiotherapy.
